# EUROPEAN PATENT APPLICATION

(11) **EP 1 652 837 A1**
(43) Date of publication of application: **03.05.2006**
(21) Application number: 04748169.2
(22) Date of filing: 27.07.2004
(51) Int. Cl.: C07C 235/66, C07C 255/53, C07C 255/54, C07C 255/55, C07C 255/57, C07C 253/20, C07C 273/18, C07C 275/54, C07D 277/66

(54) **AMINOCARBONYL NAPHTHOL DERIVATIVE, CYANONAPHTHOL DERIVATIVE, AND METHOD FOR PRODUCING THEM**

(30) Priority: 31.07.2003 JP 2003283894; 04.02.2004 JP 2004028333
(71) Applicant: Kabushiki Kaisha Ueno Seiyaku Oyo Kenkyujo, Osaka-shi, Osaka 541-0043 (JP)
(72) Inventor: UENO, Ryuzo, 6620038 (JP); KITAYAMA, Masaya, 6650881 (JP); WAKAMORI, Hiroyuki, 6693105 (JP); NISHIAKI, Miwa, 6511501 (JP); TANIKAWA, Katsunori, 6620066 (JP)
(74) Representative: Albrecht, Thomas
(86) International application number: PCT/JP2004/011014
(87) International publication number: WO 2005/012231

(57) **Abstract**

The present invention provides an aminocarbonylnaphthol derivative represented by formula (1) and process for preparing the same: 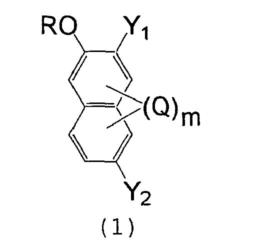 wherein each Y₁ and Y₂ is selected from the group consisting of aminocarbonyl group, carboxyl group and groups represented by formulae (2), (3) and (4), provided that at least one of Y₁ and Y₂ is aminocarbonyl group.

- (CONH) ₙ―X₁ (2)

-CO-O-X₂ (3)

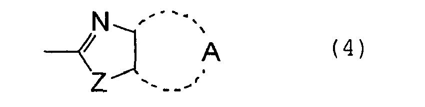

The present invention also provides a novel cyanonaphthol derivative represented by formula (7) and a salt thereof as well as process for preparing them: 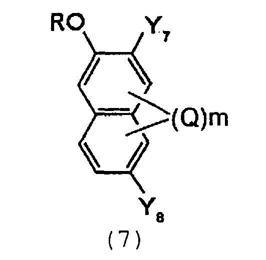 wherein each Y₇ and Y₈ is independently selected from the group consisting of cyano group, groups represented by formulae (2), (3) and (4), carboxyl group and aminocarbonyl group, provided that at least one of Y₇ and Y₈ is cyano group.

## Description

### Technical Field

The present invention relates to a novel cyanonaphthol derivative and a process for preparing the same. The present invention also relates to a novel aminocarbonylnaphthol derivative and a process for preparing the same. The aminocarbonylnaphthol derivative may be used as a synthetic intermediate of the cyanonaphthol derivative.

### Background Art

2-Naphthol derivatives are one of the most economical compounds among the condensed aromatic compounds which can form conjugated polyene systems and have adsorption in the electron band, and are easily used as raw materials for synthesis of chemicals. Therefore, they have been used for preparing various characteristic materials for example, color materials such as dyes and pigments, photosensitive materials and polymer materials such as liquid crystalline polyester.

In particular, naphthol derivatives having cyano group are useful as synthetic raw materials for liquid-crystalline materials, color materials such as dyes and pigments and biologically active agents such as medical products. Wide varieties of 2-naphthol derivatives having cyano group, for example, 6-cyano-2-naphthol derivatives which have cyano group on 6-position of naphthalene ring (Japanese Patent Applications Laid Open Nos. Sho 59-106473 and Sho 63-174963) and 3-cyano-2-naphthol derivatives which have cyano group on 3-position of naphthalene ring (Japanese Patent Application Laid Open No. Sho 63-174963) are known.

However, no cyanonaphthol derivative having substituents at both of 3- and 6-positions of 2-naphthol has yet been known.

The present inventors have found that among various 2-naphthol derivatives, 2-hydroxynaphthalene-3,6-dicarboxylic acid derivatives are particularly useful when used as coupler components for syntheses of azo compounds, because they give azo compounds which exhibit various hues and optical characteristics (Japanese Patent Nos. 3224397, 3228516, 3393869, 3393870, WO00/23525 and WO01/87859).

As coupler components for azo compounds, and in order to synthesize cyanonaphthol derivatives having substituents at both of 3- and 6-positions of 2-naphthol, novel 2-hydroxynaphthalene-3,6-dicarboxylic acid derivatives having new sets of substituents are desired.

### Disclosure of Invention

An object of the present invention is to provide a novel cyanonaphthol derivative and a process for preparing the same.

Further object of the present invention is to provide a novel naphthol derivative having aminocarbonyl group, which may be used as a synthetic intermediate for various compounds in particular for the above-mentioned cyanonaphthol derivative, as well as a process for preparing the same.

The present invention provides an aminocarbonylnaphthol derivative represented by formula (1) : 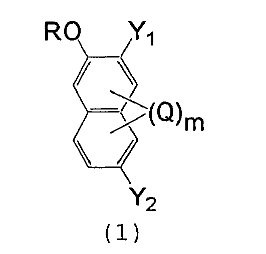 wherein each Y₁ and Y₂ is independently selected from the group consisting of aminocarbonyl group, a group represented by formula (2), a group represented by formula (3), a group represented by formula (4) and carboxyl group, provided that at least one of Y₁ and Y₂ represents aminocarbonyl group;

- (CONH) ₙ-X₁ (2)

―CO―O―X₂ (3)

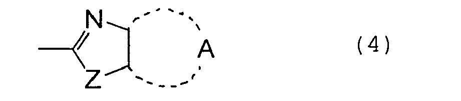

wherein n is an integer of 1 or 2;
X₁ is selected from the group consisting of an optionally branched, optionally substituted, saturated or unsaturated aliphatic group having 1-20 carbon atoms, an optionally substituted aromatic group and an optionally substituted heterocyclic group having conjugated double bonds;
X₂ is an optionally branched, saturated or unsaturated aliphatic group having 1-6 carbon atoms;
A is an optionally substituted aromatic group or an optionally substituted heterocyclic group having conjugated double bonds;
Z is selected from the group consisting of -O-, - S- and -NH-;
Q is selected from the group consisting of an optionally branched alkyl group having 1-6 carbon atoms, an optionally branched alkoxy group having 1-6 carbon atoms, halogen atom, nitro group and nitroso group;
m is an integer of 0-3;
R is selected from the group consisting of hydrogen atom, an alkaline metal, an optionally branched and optionally substituted alkyl group having 1-20 carbon atoms, an optionally branched and optionally substituted acyl group having 2-20 carbon atoms and phenylalkyl group.

Among the aminocarbonylnaphthol derivatives represented by formula (1), the compounds of which R is selected from the group consisting of an optionally branched and optionally substituted alkyl group having 1-20 carbon atoms and phenylalkyl group are preferably used as synthetic intermediates for various compounds. Such aminocarbonylnaphthol derivatives may be suitably used as synthetic intermediates for cyanonaphthol derivatives described below.

The present invention also provides a process for preparing an aminocarbonylnaphthol derivative represented by formula (6) comprising converting a carboxyl group of a naphthol derivative having carboxyl group represented by formula (5) to an acid halide and reacting the resulting acid halide of the naphthol derivative with ammonia: 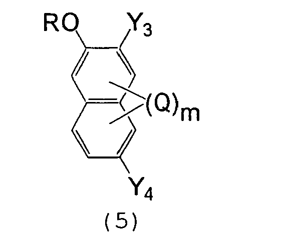 wherein each Y₃ and Y₄ is independently selected from the group consisting of carboxyl group and groups represented by formulae (2), (3) and (4), provided that at least one of Y₃ and Y₄ is carboxyl group; R, Q and m are the same as defined above; 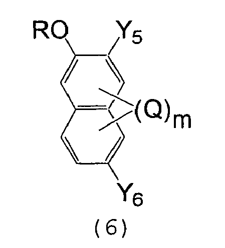 wherein each Y₅ and Y₆ is independently selected from the group consisting of aminocarbonyl group and groups represented by formulae (2), (3) and (4) defined above, provided that at least one of Y₅ and Y₆ is aminocarbonyl group; R, Q and m are the same as defined above.

Furthermore, the present invention provides a cyanonaphthol derivative represented by formula (7) and a salt thereof which may be prepared using the above-described aminocarbonylnaphthol derivative as a synthetic intermediate: 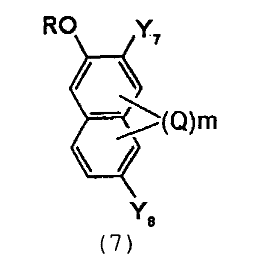 wherein each Y₇ and Y₈ is independently selected from the group consisting of cyano group, groups represented by the formulae (2), (3) and (4) defined above, carboxyl group and aminocarbonyl group, provided that at least one of Y₇ and Y₈ is cyano group; Q, R and m are the same as defined above.

The present invention also provides a cyanonaphthol derivative represented by formula (8) and a salt thereof: 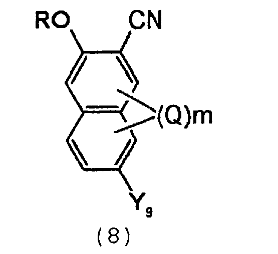 wherein Y₉ is selected from the group consisting of cyano group, groups represented by formulae (2), (3) and (4) defined above, carboxyl group and aminocarbonyl group;
R, Q and m are the same as defined above;
The present invention further provides a process for preparing a cyanonaphthol derivative represented by formula (10) comprising converting a carboxyl group of a naphthol derivative having carboxyl group represented by formula (9) to aminocarbonyl group, and reacting the resulting naphthol derivative having aminocarbonyl group with a dehydrating agent: 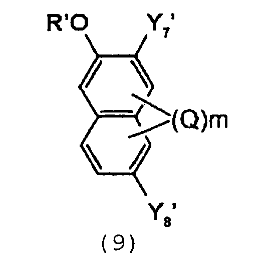 wherein each Y₇' and Y₈' is independently selected from the group consisting of carboxyl group, groups represented by formulae (2), (3) and (4) defined above, provided that at least one of Y₇' and Y₈' is carboxyl group;
R' is selected from the group consisting of an optionally branched and optionally substituted alkyl group having 1-20 carbon atoms and phenylalkyl group;
Q and m are the same as defined above; 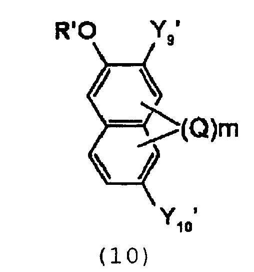 wherein each Y₉' and Y₁₀' is independently selected from the group consisting of cyano group, groups represented by formulae (2), (3) and (4) defined above, provided that at least one of Y₉' and Y₁₀' is cyano group;
R', Q and m are the same as defined above.

The dehydrating agent used in the process for preparing the cyanonaphthol derivative of the present invention is preferably phosphoryl chloride.

### Brief Description of Drawings

Figure 1 is an infrared absorption spectrum of the aminocarbonyl compound obtained in Example 1.
Figure 2 is an infrared absorption spectrum of the aminocarbonyl compound obtained in Example 2.
Figure 3 is an infrared absorption spectrum of the aminocarbonyl compound obtained in Example 3.
Figure 4 is an infrared absorption spectrum of the aminocarbonyl compound obtained in Example 4.
Figure 5 is an infrared absorption spectrum of the aminocarbonyl compound obtained in Example 5.
Figure 6 is an infrared absorption spectrum of the aminocarbonyl compound obtained in Example 6.
Figure 7 is an infrared absorption spectrum of the aminocarbonyl compound obtained in Example 7.
Figure 8 is an infrared absorption spectrum of the aminocarbonyl compound obtained in Example 8.
Figure 9 is an infrared absorption spectrum of the aminocarbonyl compound obtained in Example 9.
Figure 10 is an infrared absorption spectrum of the aminocarbonyl compound obtained in Example 10.
Figure 11 is an infrared absorption spectrum of the aminocarbonyl compound obtained in Example 11.
Figure 12 is an infrared absorption spectrum of the aminocarbonyl compound obtained in Example 12.
Figure 13 is an infrared absorption spectrum of the aminocarbonyl compound obtained in Example 13.
Figure 14 is an infrared absorption spectrum of the aminocarbonyl compound obtained in Example 14.
Figure 15 is an infrared absorption spectrum of the aminocarbonyl compound obtained in Example 15.
Figure 16 is an infrared absorption spectrum of the aminocarbonyl compound obtained in Example 16.
Figure 17 is an infrared absorption spectrum of the aminocarbonyl compound obtained in Example 17.
Figure 18 is an infrared absorption spectrum of the aminocarbonyl compound obtained in Example 18.
Figure 19 is an infrared absorption spectrum of the aminocarbonyl compound obtained in Example 19.
Figure 20 is an infrared absorption spectrum of the compound of formula [I].
Figure 21 is an infrared absorption spectrum of the compound of formula [II].
Figure 22 is an infrared absorption spectrum of the compound of formula [III].
Figure 23 is an infrared absorption spectrum of the compound of formula [IV].
Figure 24 is an infrared absorption spectrum of the compound of formula [V].
Figure 25 is an infrared absorption spectrum of the compound of formula [VI].
Figure 26 is an infrared absorption spectrum of the compound of formula [VII].
Figure 27 is an infrared absorption spectrum of the compound of formula [VIII].
Figure 28 is an infrared absorption spectrum of the compound of formula [IX].
Figure 29 is an infrared absorption spectrum of the compound of formula [X] .
Figure 30 is an infrared absorption spectrum of the compound of formula [XI].
Figure 31 is an infrared absorption spectrum of the compound of formula [XII].
Figure 32 is an infrared absorption spectrum of the compound of formula [XIII].
Figure 33 is an infrared absorption spectrum of the compound of formula [XIV].

### Best Mode for Carrying Out the Invention

In the specification and claims attached herewith, the term "aromatic group" represents a 6-membered monocyclic aromatic group or condensed ring group consisting of up to 4 of the condensed aromatic rings.

"Heterocyclic group having conjugated double bonds" represents a 5- or 6-membered monocyclic group or condensed ring group having at least one heteroatom selected from N, S and O and conjugated double bonds. When it constitutes a condensed ring group, said group may have up to 6 rings.

In an aminocarbonylnaphthol derivative represented by formula (1) and a cyanonaphthol derivative represented by formula (7) of the present invention of which Y₁, Y₂, Y₇ or Y₈ is represented by formula (2), examples of the Y₁, Y₂, Y₇ or Y₈ include alkylaminocarbonyl group, naphthylaminocarbonyl group, phenylaminocarbonyl group and the like. The aromatic group and aliphatic group of these groups may have further substituents such as halogen atom, halogenated C₁₋₆ alkyl group, nitro group, C₁₋₆ alkyl group, C₁₋₆ alkoxy group and cyano group.

In the above-formula (2) of which X₁ is an optionally substituted aromatic group, examples of the X₁ include benzene ring, naphthalene ring, anthraquinone ring and the like. In the above-formula (2) of which X₁ is an optionally substituted heterocyclic group having conjugated double bonds, examples of the X₁ include thiophene, furan, pyrrole, imidazole, pyrazole, isothiazole, isoxazole, pyridine, pyrazine, pyrimidine, pyridazine, triazole, tetrazole, indole, 1H-indazole, purine, 4H-quinolizine, isoquinoline, quinoline, phthalazine, naphthyridine, quinoxaline, quinazoline, cinnoline, pteridine, benzofuran and the like.

Examples of substituents on X₁ include halogen atom, halogenated C₁₋₆ alkyl, nitro group, C₁₋₆ alkyl group, C₁₋₆ alkoxy group (such as methoxy group), cyano group, phenoxy group, pyrimidylamino group, benzoylamino group, sulfonic acid group, esterified carboxyl group (such as alkoxycarbonyl group, phenoxycarbonyl group), amidated carboxyl group (such as phenylaminocarbonyl group), alkylaminosulfonyl group, C₂₋₆ alkenyl group optionally having aryl group and the like.

The above substituents may be in the form of salts with alkaline metals.

When the substituent on X₁ contains aromatic ring group, said ring may have one or more further substituents such as halogen atom, C₁₋₆ alkyl group, C₁₋₆ alkoxy group, phenyl group, cyano group and the like.

In the compound of the present invention of which Y₁, Y₂, Y₇ or Y₈ is a group represented by formula (3), examples of the Y₁, Y₂, Y₇ or Y₈ include methoxycarbonyl group, ethoxycarbonyl group, n-propyloxycarbonyl group, iso-propyloxycarbonyl group, n-butyloxycarbonyl group and the like.

In the compound of the present invention of which Y₁, Y₂, Y₇ or Y₈ is a group represented by formula (4), examples of the Y₁, Y₂, Y₇ or Y₈ include benzothiazolyl group, benzoxazolyl group, benzimidazolyl group and the like. Examples of optionally substituted aromatic groups which constitute ring A of formula (4) include benzene ring, naphthalene ring, anthraquinone ring and the like. Examples of optionally substituted heterocyclic groups having conjugated double bonds include thiophene, furan, pyrrole, imidazole, pyrazole, isothiazole, isoxazole, pyridine, pyrazine, pyrimidine, pyridazine, triazole, tetrazole, indole, 1H-indazole, purine, 4H-quinolizine, isoquinoline, quinoline, phthalazine, naphthyridine, quinoxaline, quinazoline, cinnoline, pteridine, benzofuran and the like.

In aminocarbonylnaphthol derivatives represented by formula (1) and aminocarbonylnaphthol derivatives represented by formulae (7) and (8) of the present invention, examples of R include hydrogen atom, alkaline metals (such as sodium and potassium), optionally branched and optionally substituted alkyl groups having 1-20 carbon atoms (such as methyl group, ethyl group, n-octyl group and n-hexadecyl group), acyl groups (such as acetyl group) and phenylalkyl groups (such as benzyl group) and the like.

In particular, among the aminocarbonylnaphthol derivatives represented by formula (1), the compounds of which R is selected from the group consisting of an optionally branched and optionally substituted alkyl group having 1-20 carbon atoms and phenylalkyl group are preferably used as synthetic intermediates of the cyanonaphthol derivatives of the present invention.

A process for preparing an aminocarbonylnaphthol derivative represented by formula (1) is further described herein below, but the process does not limit the present invention.

A preferable method for preparing a compound of formula (1) of which neither Y₁ nor Y₂ is carboxyl group comprises converting carboxyl group of a naphthol derivative having carboxyl group represented by formula (5) to acid halide and reacting the resulting acid halide with ammonia.

A preferable method for preparing a compound of formula (1) of which one of Y₁ and Y₂ is aminocarbonyl group and the other is carboxyl group comprises hydrolyzing ester group of an aminocarbonylnaphthol derivative of formula (1) of which one of Y₁ and Y₂ is aminocarbonyl group and the other is ester group represented by formula (3) by base in an aqueous solvent and subjecting the resulting product to acid crystallization.

Examples of acid halogenating agents used for the acid halogenation include thionyl chloride, thionyl bromide, oxalyl chloride and the like. Examples of solvents used for the acid halogenation include xylene, toluene, tetrahydrofuran and the like.

The temperature of the reaction of a naphthol derivative having carboxyl group represented by formula (5) with an acid halogenating agent is preferably no more than 80°C and is more preferably 30-50°C.

After the completion of the acid halogenation, the solvent and the excess acid halogenating agent may be removed by means such as distilling them away under reduced pressure to isolate acid halide. The resulting acid halide may be reacted with ammonia in the same solvent as that used for acid halogenation. Alternatively, after the completion of the acid halogenation and the removal of excess acid halogenating agent, acid halide may be directly reacted with ammonia.

An aminocarbonylnaphthol derivative represented by formula (1) of which R is alkyl group, acyl group or phenylalkyl group may be prepared by conventional alkylation, acylation or phenyl alkylation of hydroxyl group of the aminocarbonylnaphthol- derivative represented by formula (1) of which R is hydrogen atom. An aminocarbonylnaphthol derivative represented by formula (1) of which R is an alkaline metal may be prepared by the method comprising dissolving the aminocarbonylnaphthol derivative represented by formula (1) of which R is hydrogen atom in organic solvent such as lower alcohol and treating the solution with basic alkaline metal compound such as sodium methoxide, potassium butoxide, sodium hydroxide, sodium carbonate and the like.

A process for preparing an aminocarbonylnaphthol derivative represented by formula (1) is further described below in more detail.

An aminocarbonylnaphthol derivative of formula (1) of which both of 3- and 6- positions are aminocarbonyl groups may be prepared according to scheme 1 below. 2-hydroxynaphthalene-3,6-dicarboxylic acid derivative represented by formula (11) is reacted with thionyl chloride to give a carboxylic acid chloride of formula (12), which is reacted with ammonia to give the aminocarbonylnaphthol derivative of formula (1) of which both of 3- and 6- positions are aminocarbonyl groups.

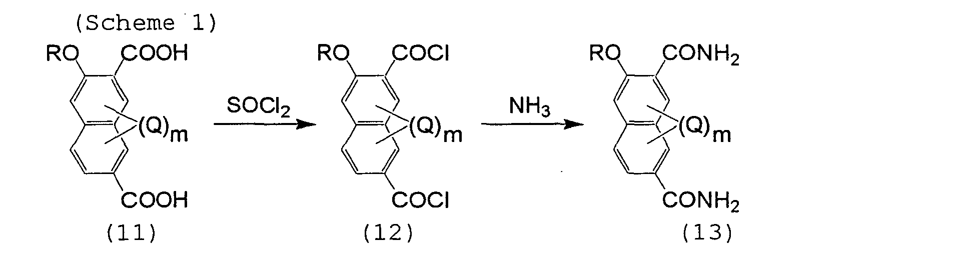

In formulae (11), (12) and (13), Q, m and R are the same as defined in formula (1).

An aminocarbonylnaphthol derivative of formula (1) of which only one of 3- or 6-position is aminocarbonyl group and the other is a group selected from the group consisting of formula (2), formula (3) and formula (4), for example, an aminocarbonylnaphthol derivative of formula (1) of which only 6-position is aminocarbonyl group may be prepared according to scheme 2 below.

2-Hydroxynaphthalene-3,6-dicarboxylic acid derivative represented by formula (14) may be reacted with thionyl chloride to give carboxylic acid chloride of formula (15), which is reacted with ammonia to give a 2-hydroxynaphthalene-3,6-dicarboxylic acid derivative of formula (1) of which 6- position is aminocarbonyl group. A compound of formula (1) of which only 3-position is aminocarbonyl group may be prepared by the same manner.

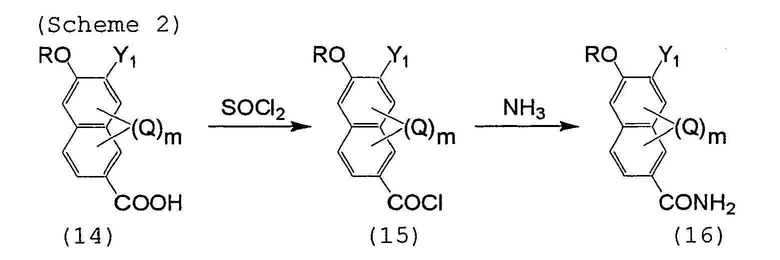

In formulae (14), (15) and (16), Y₁ is selected from the group consisting of formula (2), formula (3) and formula (4), and Q, m and R are the same as defined in formula (1).

An aminonaphthol derivative of formula (1) of which one of 3- or 6- position is aminocarbonyl group and the other is carboxyl group may be prepared from the aminocarbonylnaphthol derivative obtained in scheme 2 by the method described below.

For example, an aminocarbonylnaphthol derivative of formula (1) of which 3-position is aminocarbonyl group and 6-position is carboxyl group may be prepared according to the scheme 3 below. The ester group of an aminocarbonylnaphthol derivative represented by formula (17) of which Y₂ is formula (3) is hydrolyzed by base such as sodium hydroxide and potassium hydroxide in aqueous solvent and thus obtained product is subjected to crystallization by addition of acid such as hydrochloric acid, sulfuric acid and nitric acid to give aminocarbonylnaphthol derivative of formula (1) of which 3-position is aminocarbonyl group and 6-position is carboxyl group. An aminocarbonylnaphthol derivative of formula (1) of which 3-position is carboxyl group may be prepared by the same manner.

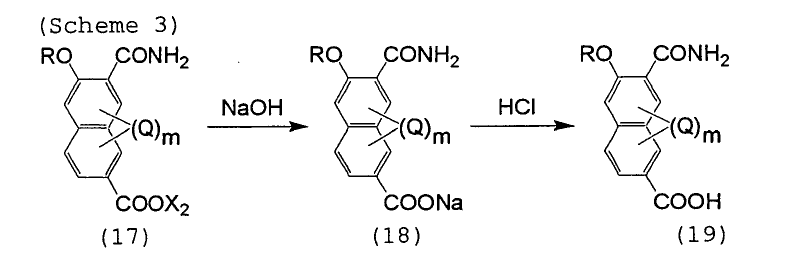

Processes for preparing 2-Hydroxynaphthalene-3,6-dicarboxylic acid derivatives of formula (11), formula (14) and formula (17) which are the starting compounds of the scheme 1, scheme 2 and scheme 3 are not limited, but the methods disclosed in WO96/32366 and WO01/87859 may be employed.

The aminocarbonyl group of thus obtained aminocarbonylnaphthol derivative may be converted to cyano group by dehydration reaction and the like to obtain a cyanonaphthol derivative. In other words, the aminocarbonylnaphthol derivatives of the present invention are useful synthetic intermediates for various novel 2-naphthol derivatives.

A process for preparing a cyanonaphthol derivative represented by formula (7) is further described herein below, but the process does not limit the present invention.

A cyanonaphthol derivative of the present invention of which R is alkyl group or phenylalkyl group, and both of Y₇ and Y₈ are cyano group may be prepared from a naphthol derivative represented by formula (20) according to scheme 4 below.

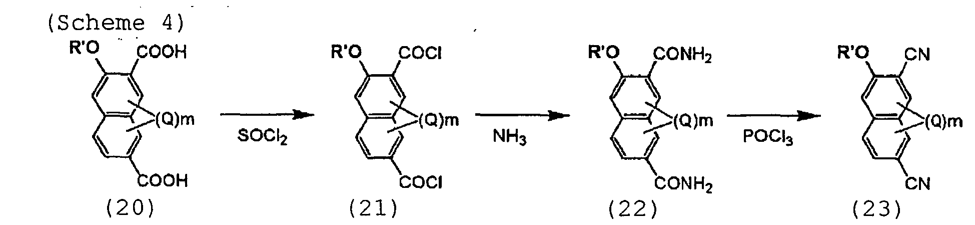

In formulae (20), (21), (22) and (23), R' is selected from the group consisting of an optionally branched and optionally substituted alkyl group having 1-20 carbon atoms and phenylalkyl group; Q and m are the same as defined in formula (1).

Specifically, carboxyl group of a compound represented by formula (20) is converted to chlorocarbonyl group by reaction with thionyl chloride and the like in a solvent selected from tetrahydrofuran, xylene and toluene to give a compound represented by formula (21). Then the compound of formula (21) is reacted with ammonia to give a naphthol derivative having aminocarbonyl group represented by formula (22). The aminocarbonyl group of the resulting naphthol derivative of formula (22) is converted to cyano group by the following procedure. The naphthol derivative of formula (22) is reacted with dehydrating agent selected from phosphoryl chloride, phosphorous trichloride, phosphorus pentoxide, phosphorous pentachloride, thionyl chloride, p-toluenesulfonyl chloride, benzenesulfonyl chloride, N,N'-dicyclohexylcarbodiimide, cyclohexane-1,2-dicarboxylic acid anhydride and 2-chlorobenzoxazolium salt in solvent such as o-dichlorobenzene, xylene, mesitylene and diethylbenzene at temperatures of 50-200°C, preferably at 80-160°C to give a cyanonaphthol derivative represented by formula (23).

An especially preferable dehydrating agent is phosphoryl chloride.

A cyanonaphthol derivative of the present invention represented by formula (7) of which R is alkyl group or phenylalkyl group, and only one of Y₇ or Y₈ is cyano group and the other is a group represented by formula (2), formula (3) or formula (4) may be prepared from a naphthol derivative represented by formula (24) or (28) according to the scheme 5 or scheme 6 below:

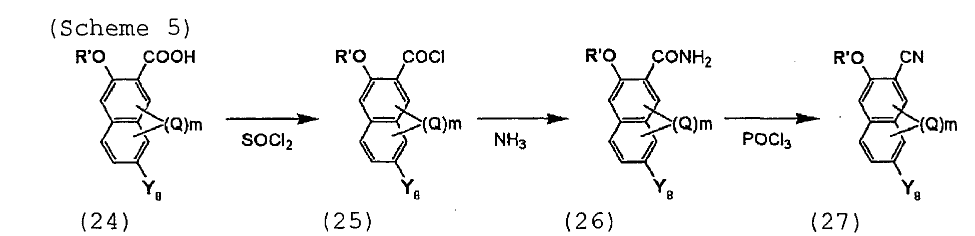

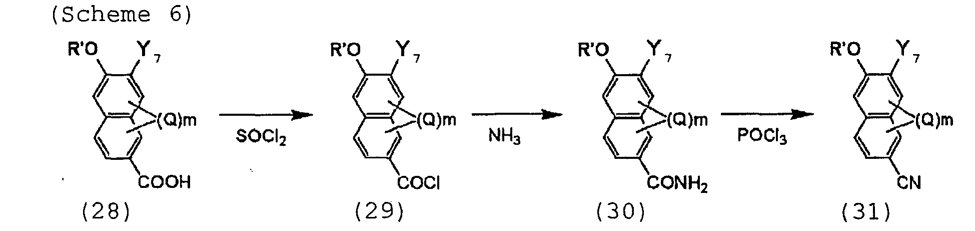

In formulae (24)-(31) of scheme 5 and scheme 6, R' is selected from the group consisting or an optionally branched and optionally substituted alkyl group having 1-20 carbon atoms and phenylalkyl group, each Y₇ and Y₈ is selected from the group consisting of formula (2), formula (3) and formula (4), and Q, and m are the same as defined in formula (1).

In scheme 4 to 6, naphthol derivatives represented by formula (20), formula (24) and formula (28), from which cyanonaphthol derivatives are synthesized, may be prepared by methods described in WO96/32366 and WO01/87859.

A cyanonaphthol derivative of the present invention represented by formula (7) of which R is hydrogen atom may be prepared by reacting a cyanonaphthol derivative represented by formula (23), formula (27) or formula (31) which is obtained in the scheme 4 to 6 with aluminum chloride, hydrobromic acid and the like.

A cyanonaphthol derivative of the present invention represented by formula (7) of which R is acyl group may be prepared by reacting a cyanonaphthol derivative represented by formula (7) of which R is hydrogen atom with acylating agent selected from acetic anhydride, propionic anhydride, pivalic anhydride and the like.

A salt of cyanonaphthol derivative represented by formula (7) of the present invention of which R is an alkaline metal may be prepared by reacting a cyanonaphthol derivative represented by formula (7) of which R is hydrogen atom with a basic alkaline metal compound such as an alkaline metal hydroxide (for example, sodium hydroxide), an alkaline metal carbonate (for example, sodium carbonate) and an alkaline metal alkoxide (for example, sodium methoxide).

Preferable alkaline metals are sodium, potassium and lithium. Among them, sodium and potassium are especially preferable.

A cyanonaphthol derivative of the present invention represented by formula (7) of which one of Y₇ and Y₈ is carboxyl group may be prepared by hydrolyzing a cyanonaphthol derivative represented by formula (7) of which one of Y₇ and Y₈ is carboxylic ester of formula (3) under heating in solvent selected from alcohols such as methanol, ethanol, isopropanol, n-butanol and aqueous solution of these alcohols in the presence of base such as sodium hydroxide and subjecting the reaction mixture to acid crystallization using hydrochloric acid and the like.

When at least one of Y₇ and Y₈ is carboxyl group, the carboxyl group may be present as an alkaline metal salt. Methods for preparing alkaline metal salts and examples of preferable alkaline metals are the same as those described for R of cyanonaphthol derivatives represented by formula (7).

A cyanonaphthol derivative of the present invention represented by formula (7) of which one of Y₇ and Y₈ is aminocarbonyl group may be prepared by converting carboxyl group of the cyanonaphthol derivative represented by formula (7) of which one of Y₇ and Y₈ is carboxyl group to aminocarbonyl group by conventional methods such as reaction of carboxylic acid chloride with ammonia.

A cyanonaphthol derivative of the present invention represented by formula (7) of which one of Y₇ and Y₈ is a group selected from formula (2), formula (3) and formula (4) may be prepared according to the method of scheme 5 or scheme 6. Alternatively, a cyanonaphthol derivative of formula (7) of which one of Y₇ and Y₈ is a group selected from formula (2), formula (3) and formula (4) may be prepared by converting carboxyl group of a cyanonaphthol derivative of formula (7) of which one of Y₇ and Y₈ is carboxyl group into a group selected from formula (2), formula (3) and formula (4) according to the methods described in WO96/32366 and WO01/87859.

### Industrial Applicability

An aminocarbonylnaphthol derivative of the present invention may be used as it is or as an intermediate for synthesizing various organic compounds. The various naphthol derivatives synthesized from the aminocarbonylnaphthol derivative of the present invention may be used as couplers of azo compounds which exhibit various colors and excellent properties such as weather resistance.

In addition, a cyanonaphthol derivative of the present invention may be suitably used as synthetic raw material of color materials such as azo color and dyes and pigments comprising diketopyrrolopyrrole, of liquid crystal materials as well as of polymer materials such as liquid crystalline polyesters.

Moreover, since a cyanonaphthol derivative of the present invention has substituents at both 3- and 6-positions, it may allow synthesis of color materials with various hues, and in particular, it may be suitably used as synthetic raw material for color materials such as dyes and pigments.

The syntheses of aminocarbonylnaphthol derivatives of the present invention are further illustrated by the following examples 1-19.

### Example 1

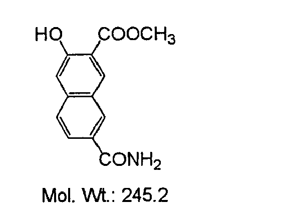

148 g of 3-hydroxy-2-methoxycarbonyl-7-naphthoic acid was dissolved in 1100 g of THF and to this solution, 0.5 g of N,N-dimethylformamide and 143 g of thionyl chloride were added sequentially and the mixture was reacted for two hours at 50°C. From the reaction mixture, excess thionyl chloride was distilled away. To this reaction mixture, 1100 g of THF was added to give a solution. To this solution, ammonia gas was added and the mixture was reacted at 40°C for 2 hours. After cooling the reaction mixture to room temperature, precipitate was filtrated, washed well with water and methanol and dried to give 126 g of the intended compound as white powder (decomposition point: 203°C, mass analysis :m/z(-) 244).

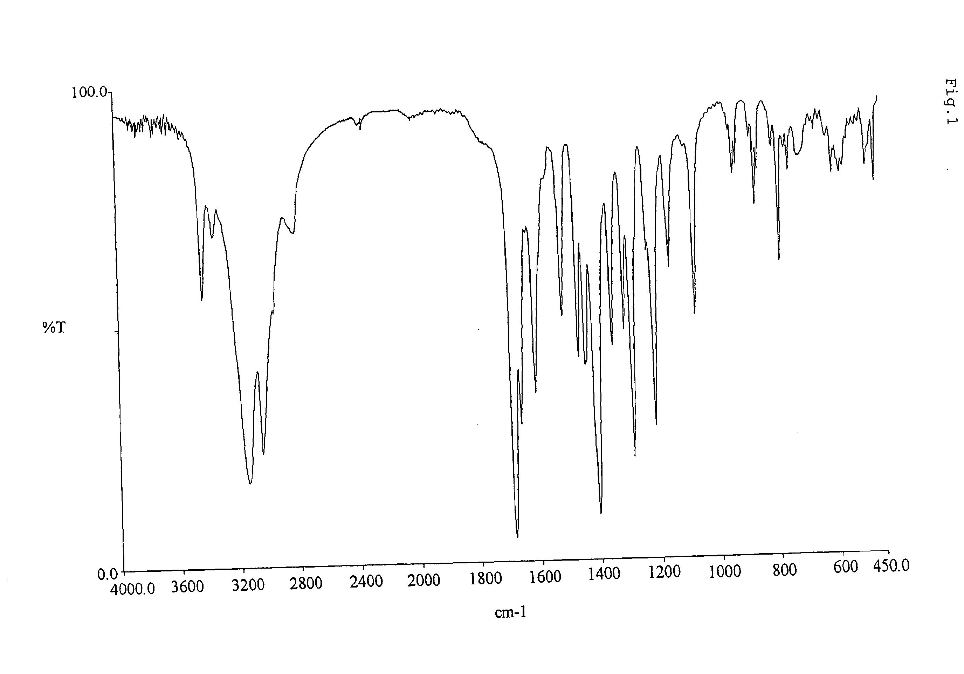

The infrared absorption spectrum (KBr method) of this compound is shown in Figure 1.

### Examples 2-18

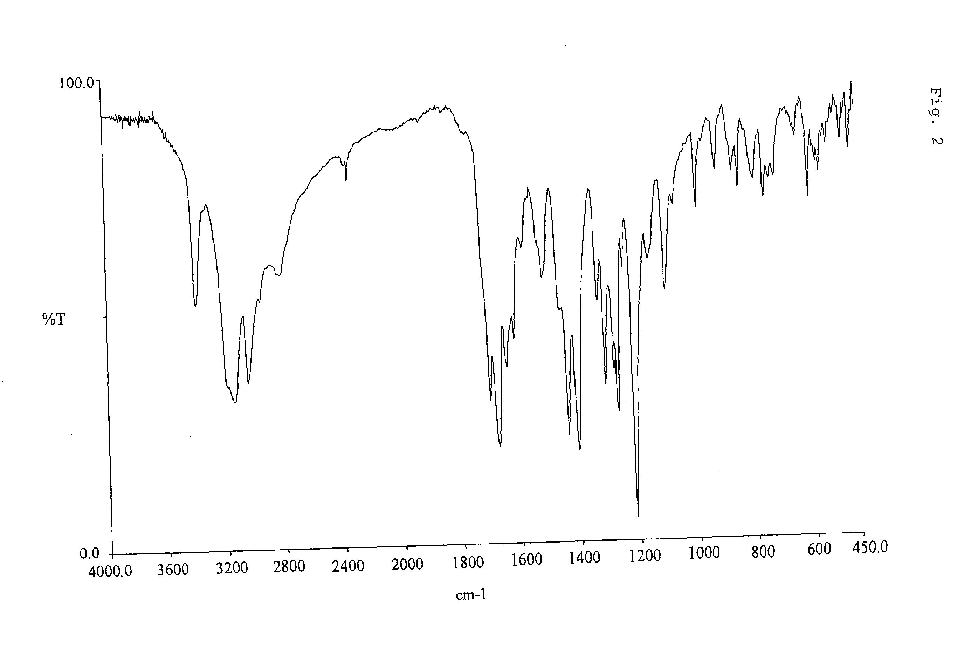

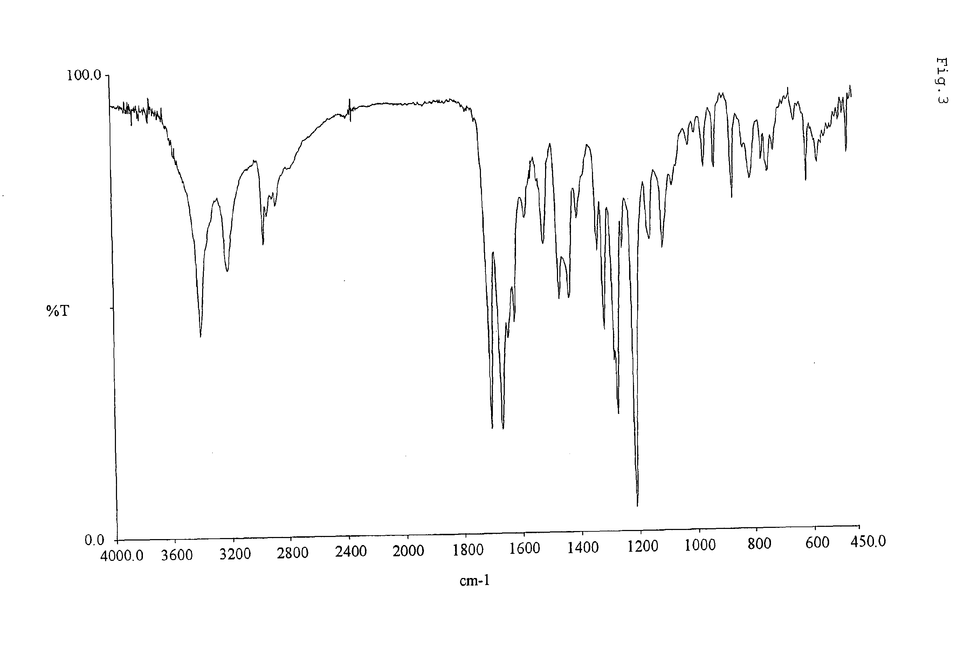

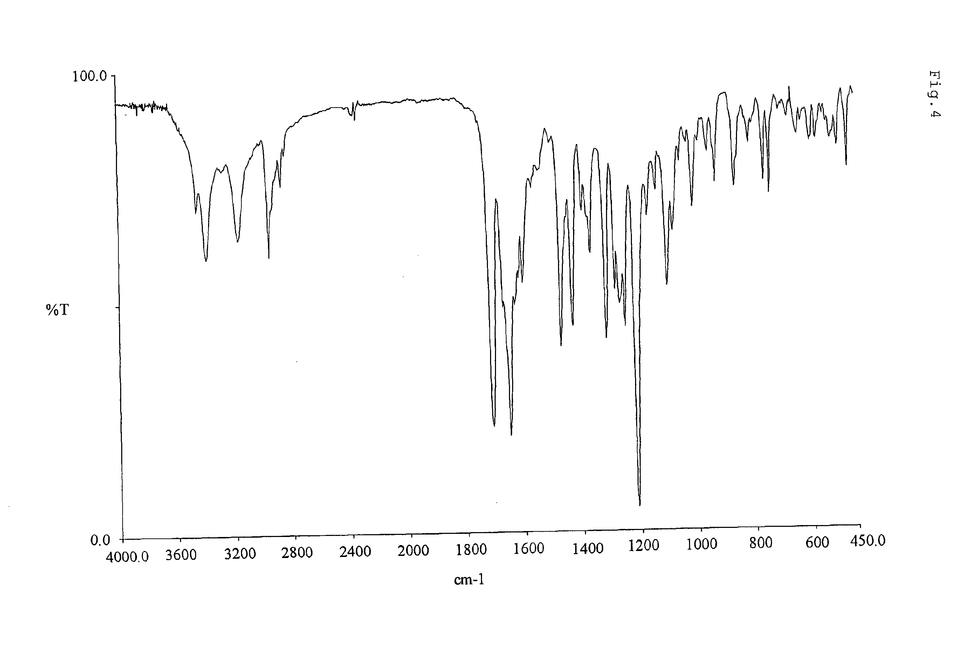

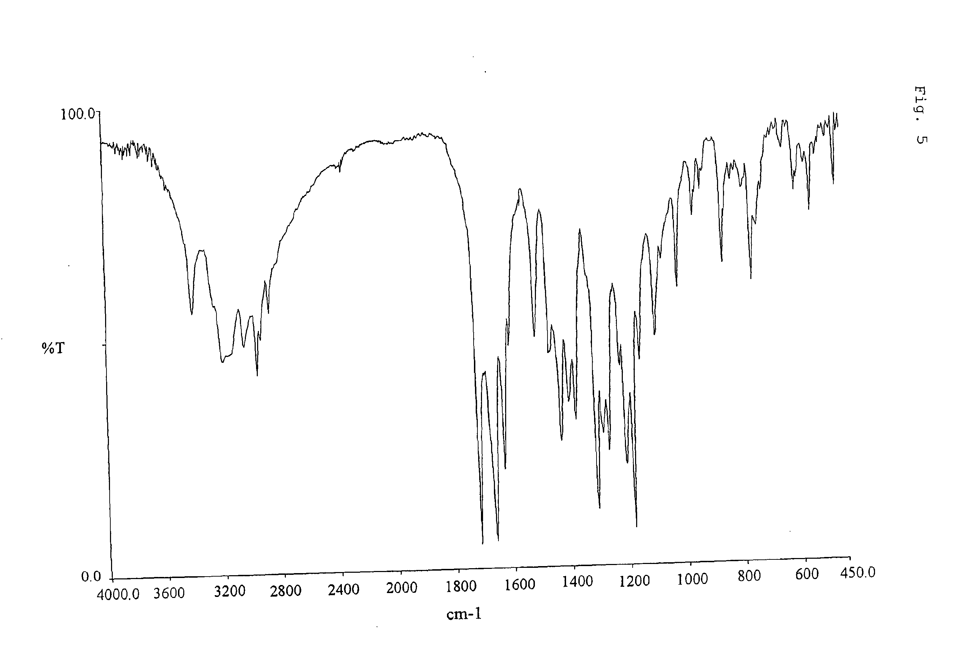

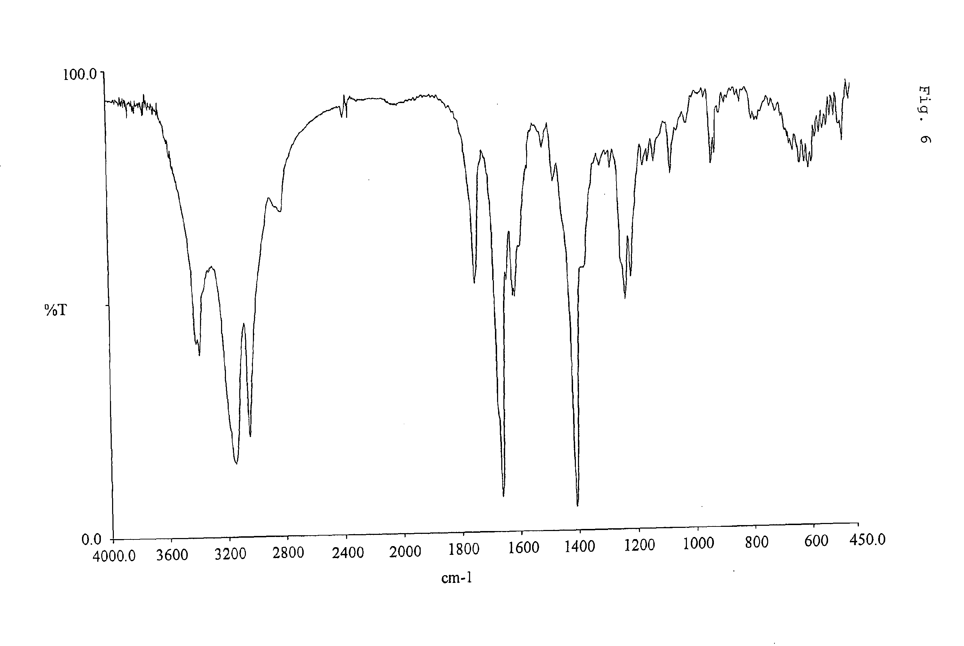

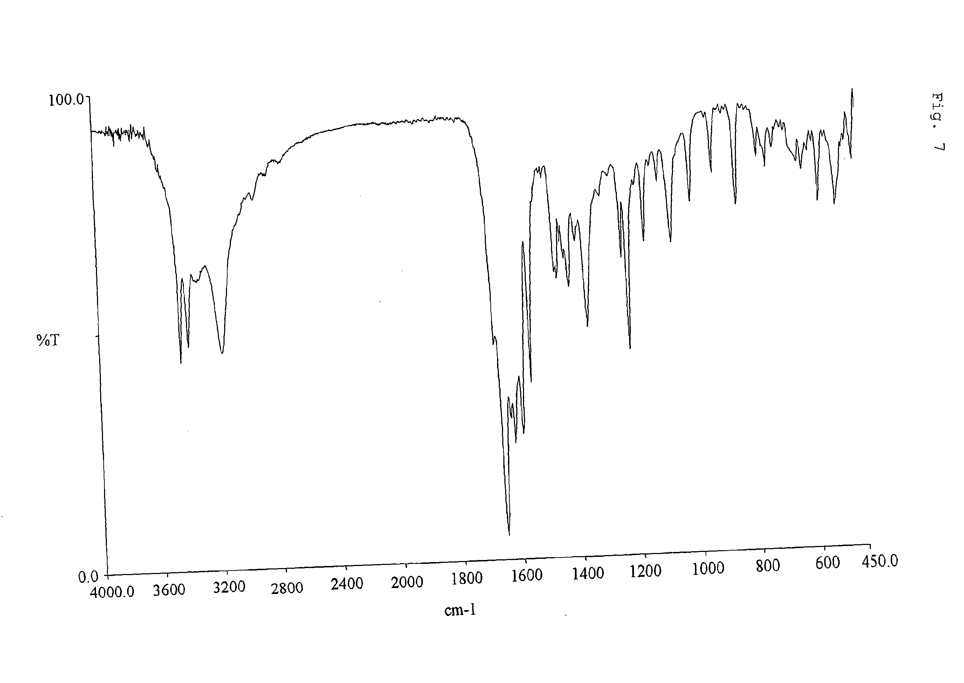

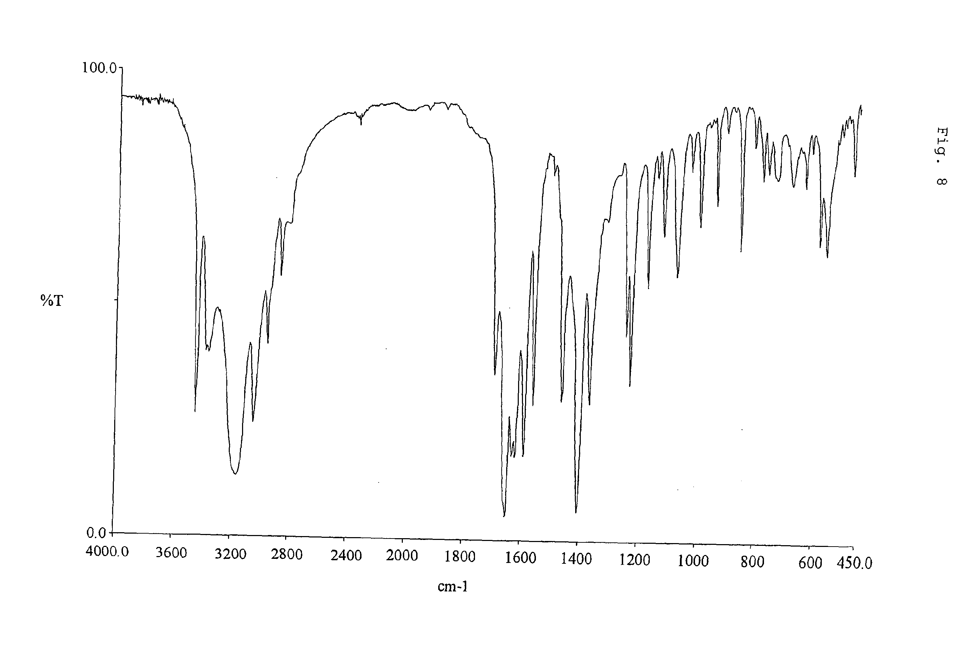

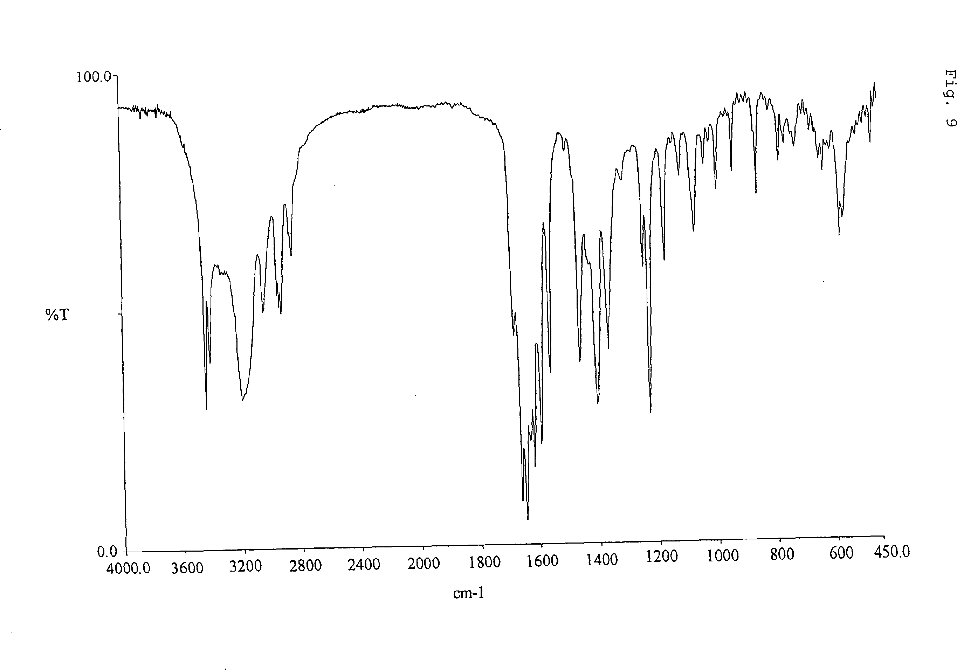

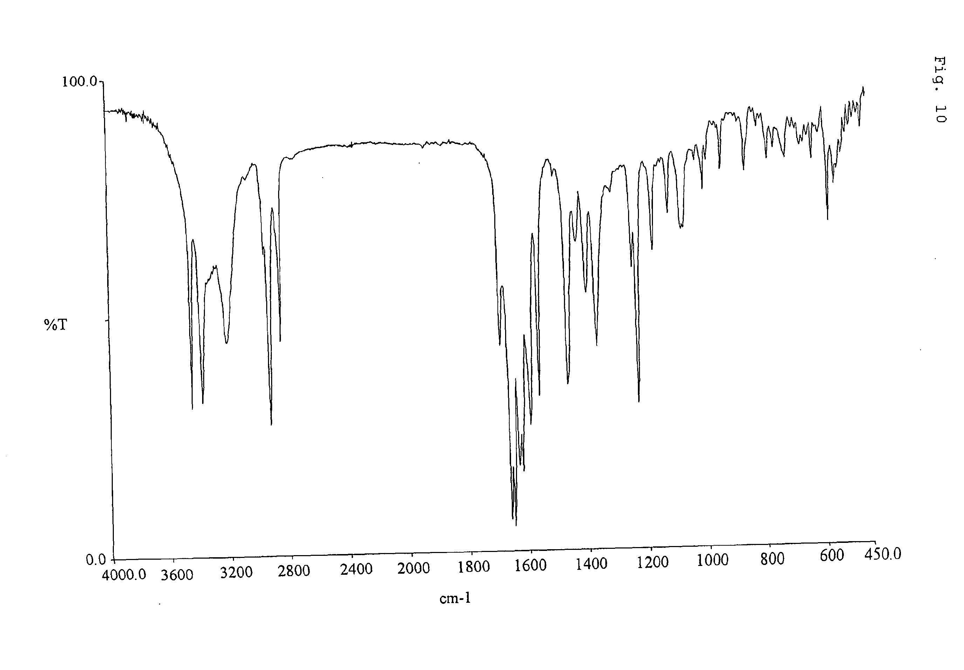

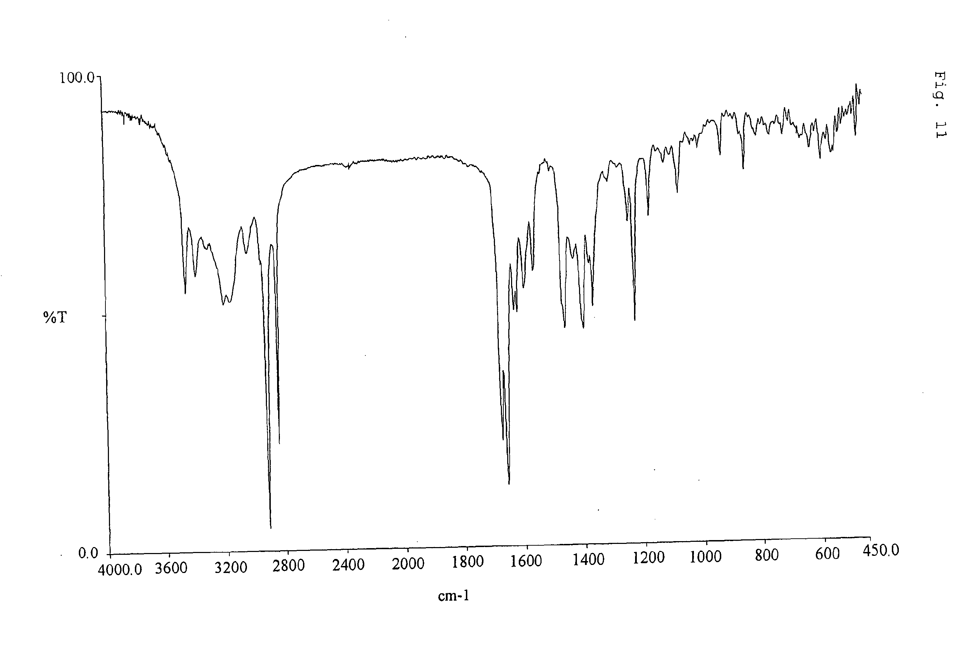

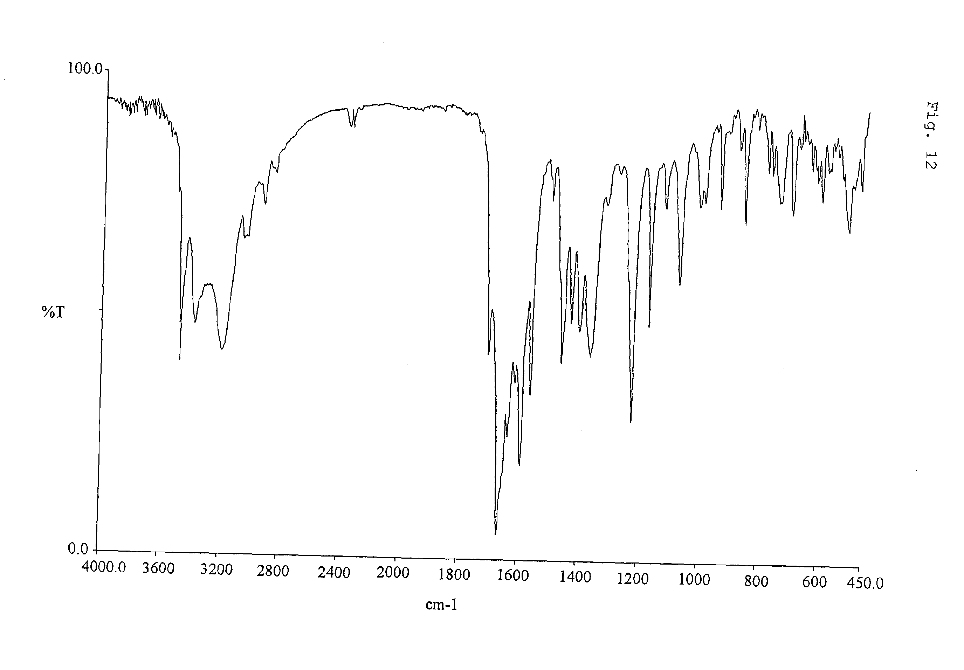

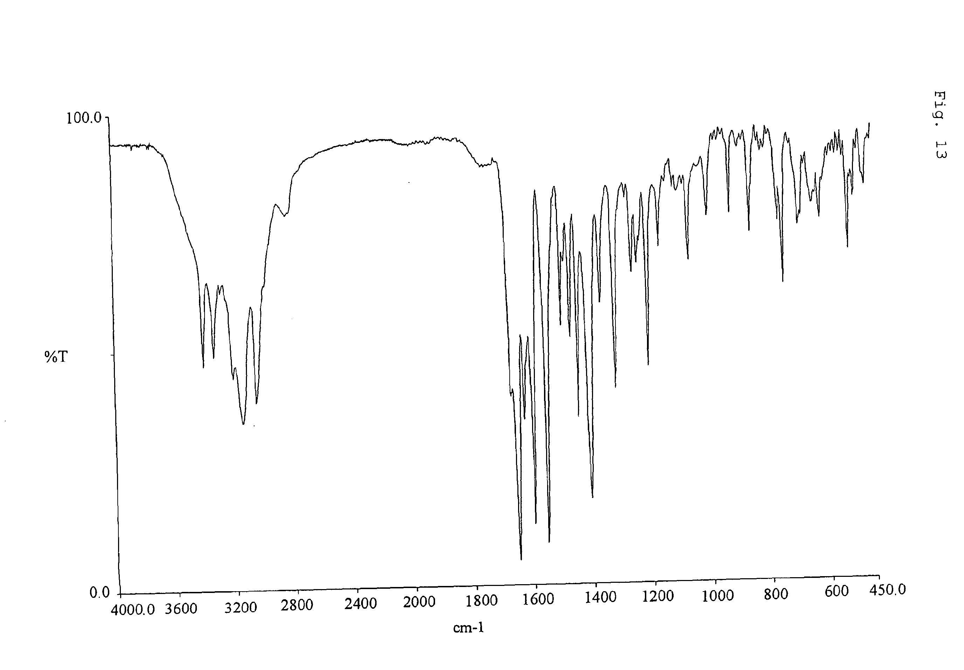

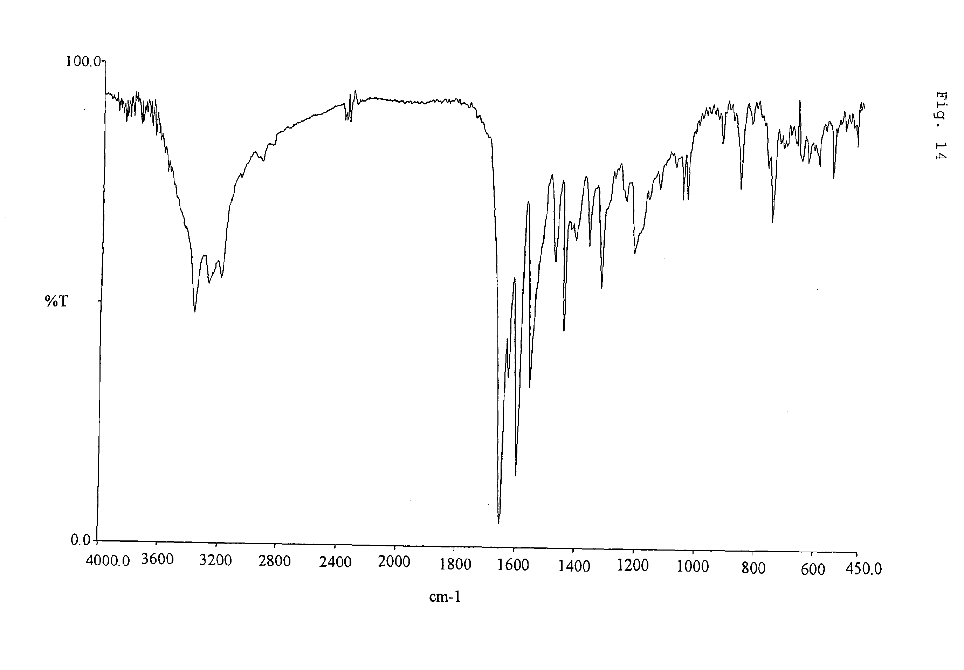

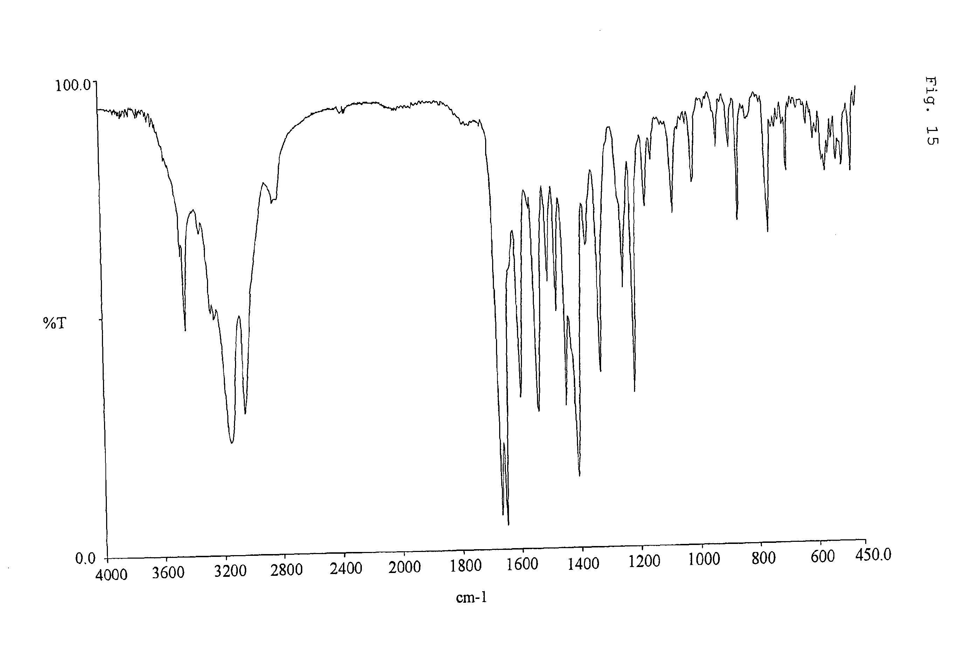

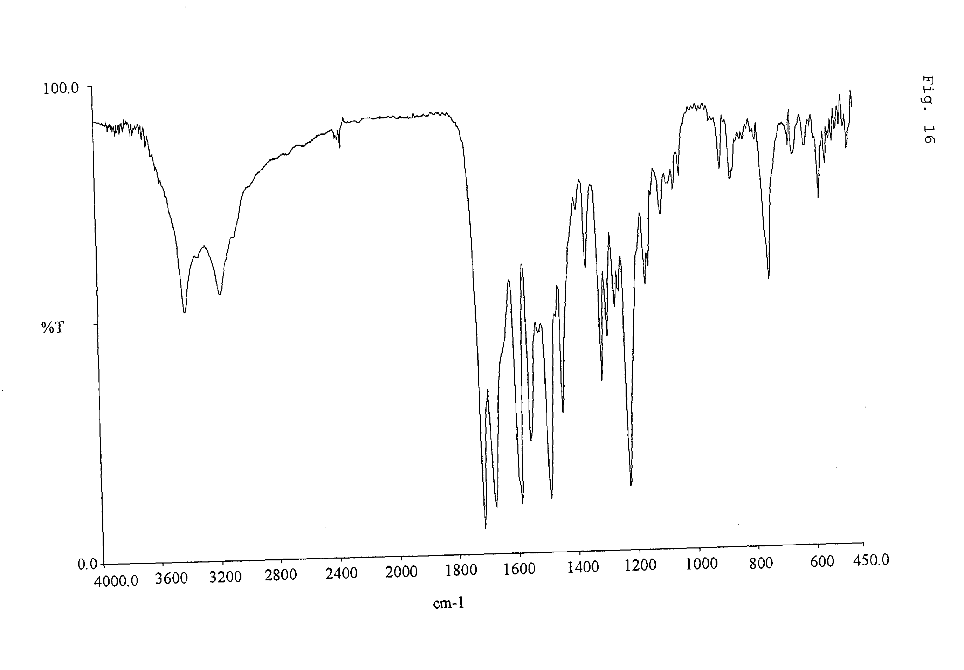

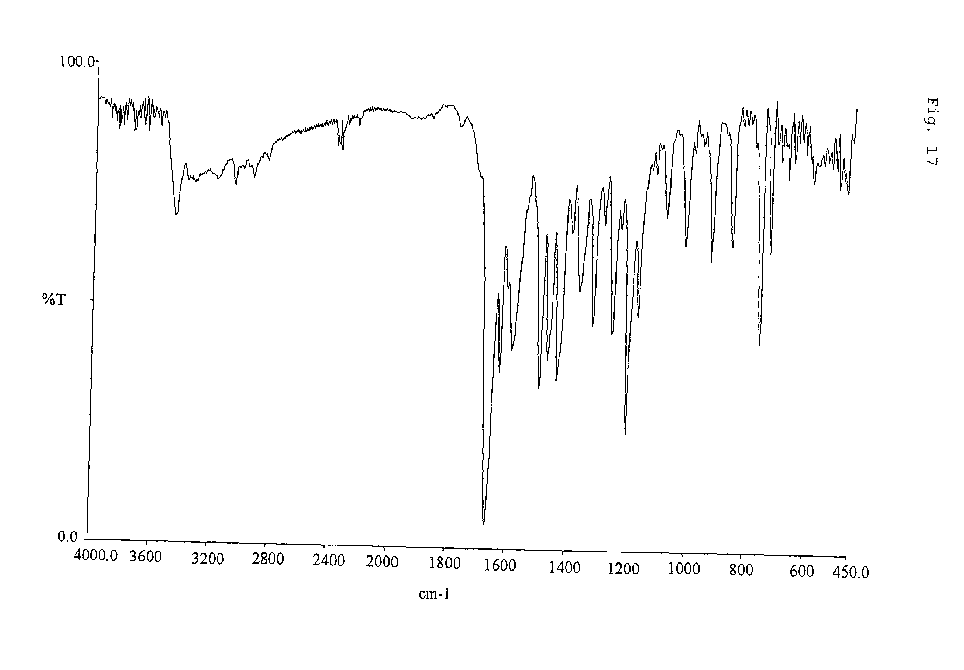

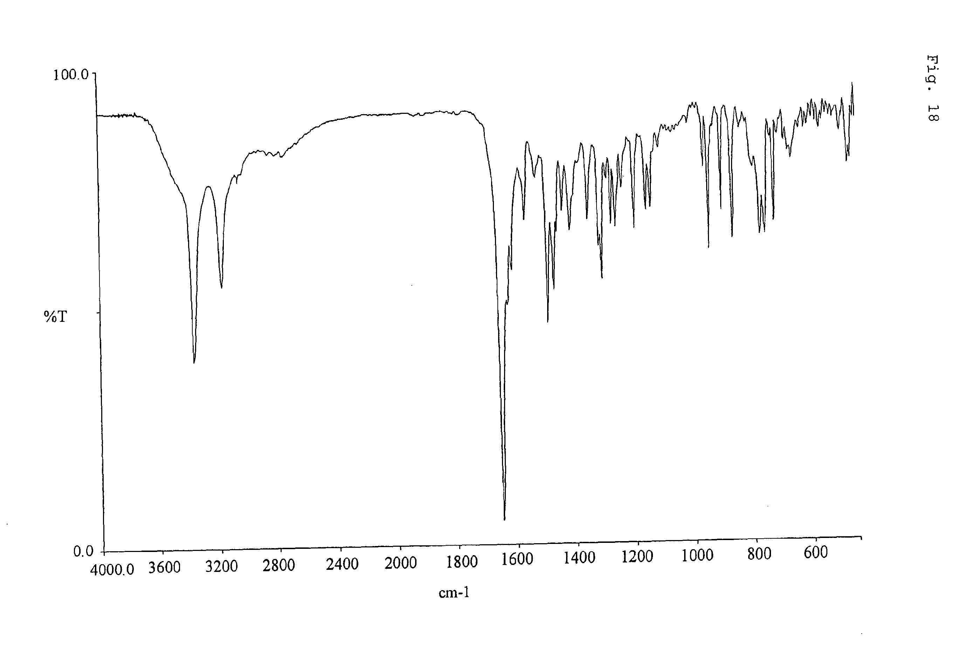

Aminocarbonyl compounds were synthesized according to the same manner as described in Example 1 with the exception that carboxylic acids shown in Table 1 were used instead of 3-hydroxy-2-methoxycarbonyl-7-naphthoic acid, provided that when carboxylic acid equivalent was two, thionyl chloride used was changed to two-fold equivalent. The melting points, decomposition points and results of mass analyses of the synthesized aminocarbonyl compounds were shown in Table 1. The infrared absorption spectra (KBr method) of these compounds are shown in Figures 2 to 18.

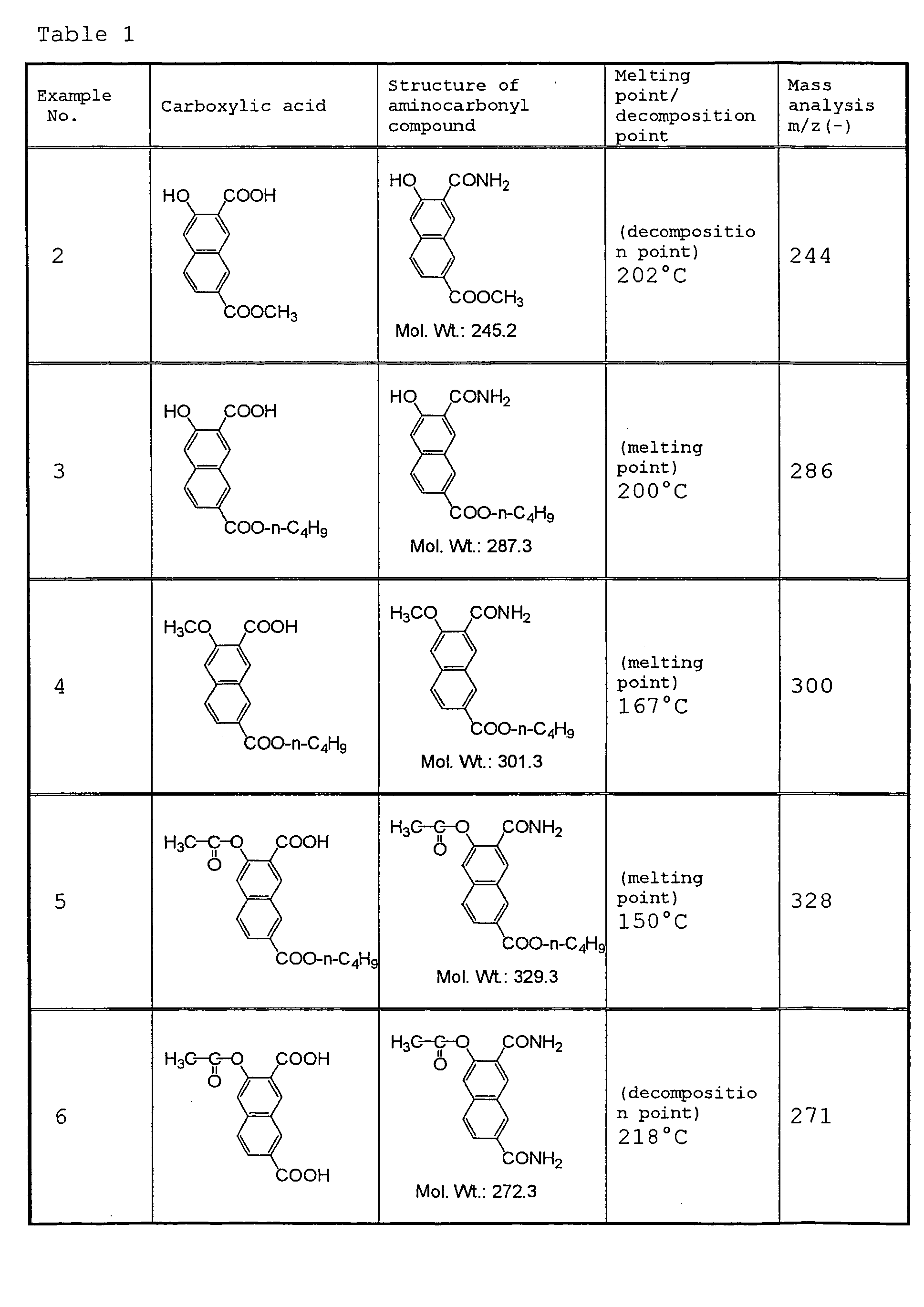

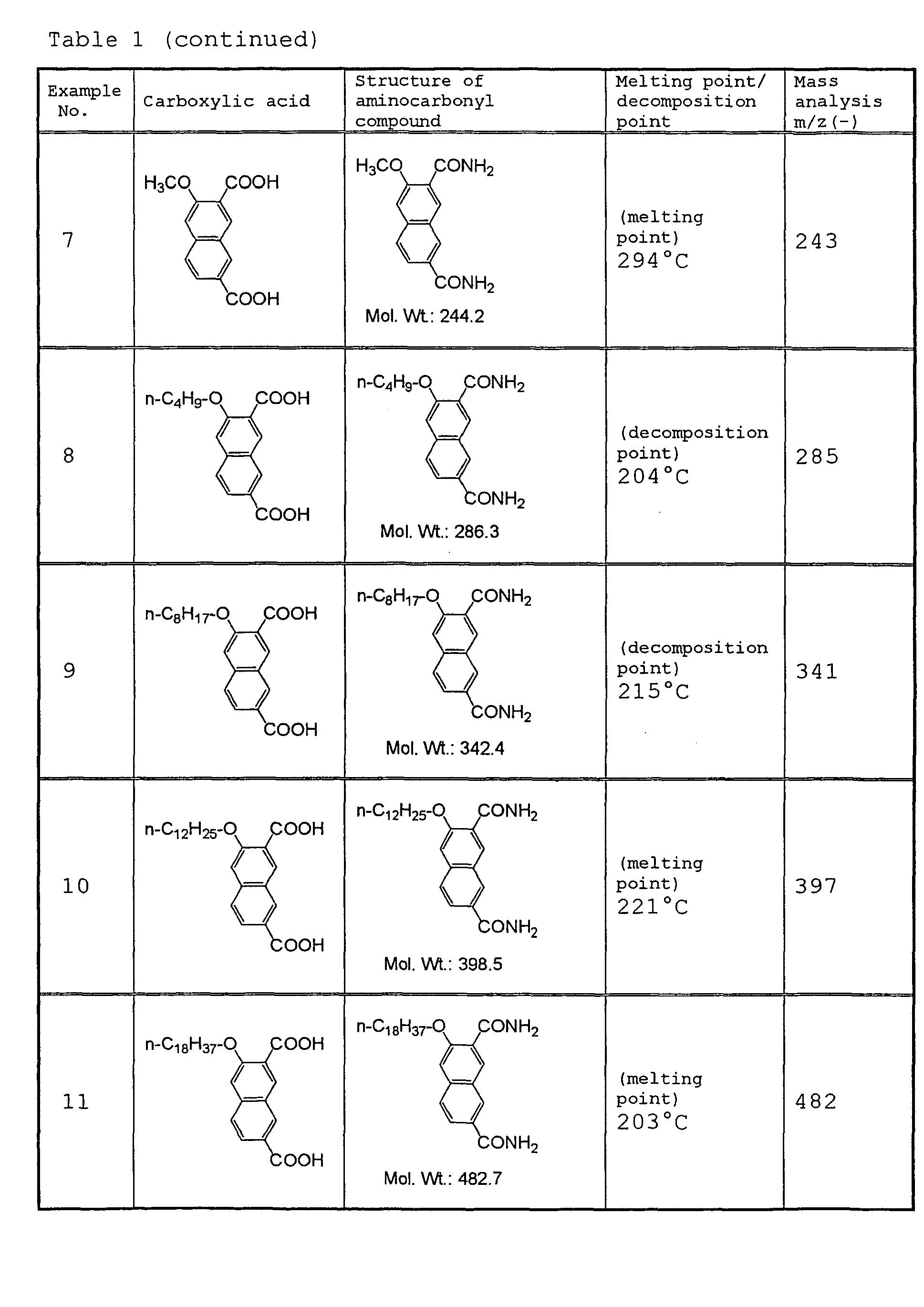

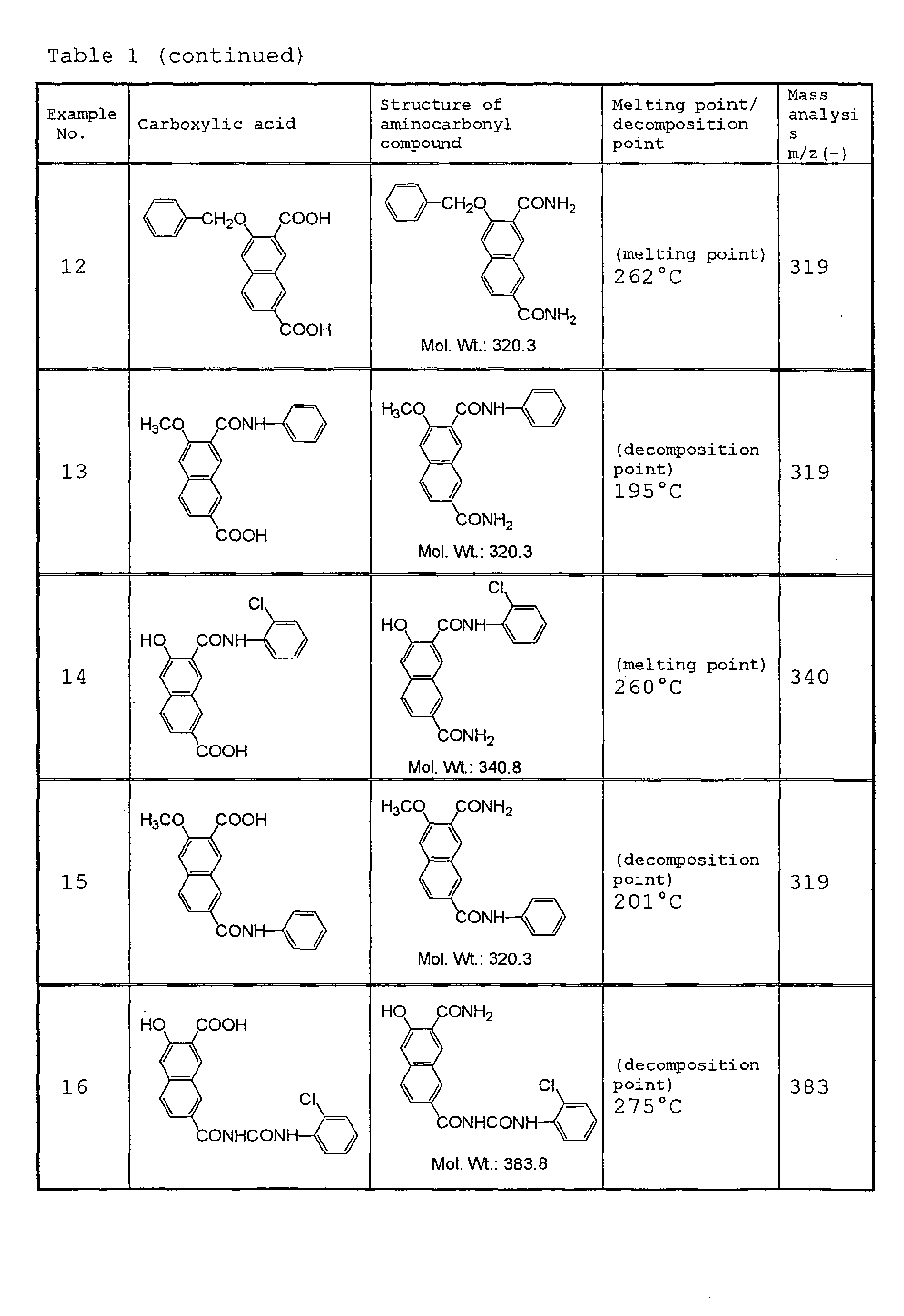

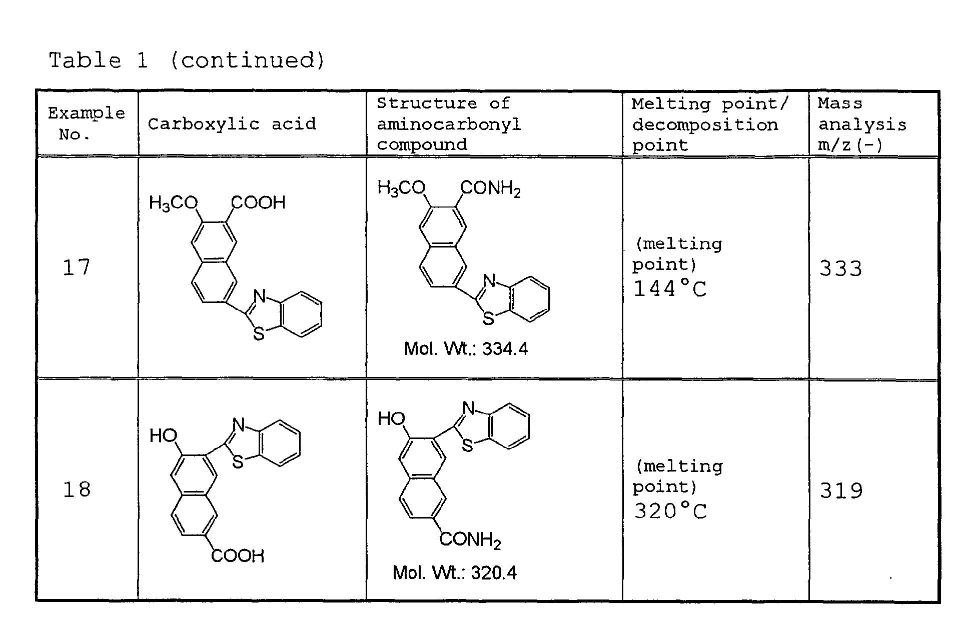

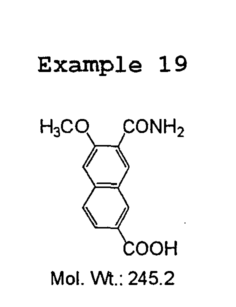

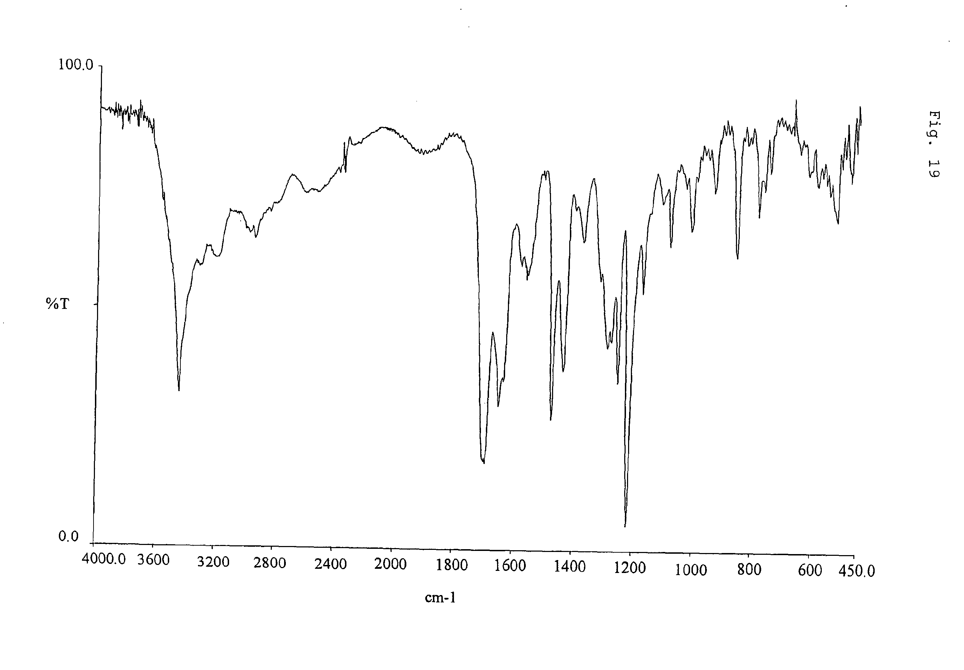

18.0 g of 3-aminocarbonyl-2-methoxy-6-n-butoxycarbonylnaphthalene obtained in Example 4 was suspended in 50 g of methanol and 20 g of water. To this suspension, 2.5 g of sodium hydroxide was added and the mixture was reacted at 60°C for two hours. After insoluble matter was removed, the pH of the reaction mixture was adjusted to pH 2 by means of 10 % hydrochloric acid and then the precipitated crystal was filtrated. The crystal was washed well with water and dried to give 12.9 g of the intended compound as yellow powder. (Melting point: 246°C, decomposition point: 286°C, mass analysis: m/z (-) 244). The infrared absorption spectrum (KBr method) of this compound is shown in Figure 19.

The syntheses of cyanonaphthol derivatives of the present invention were further illustrated in detail by the following Examples 20 to 33.

### Example 20

### Synthesis of 2-methoxy-3,6-dicyanonaphthalene

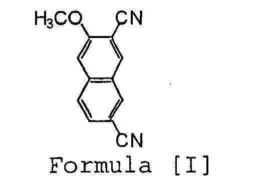

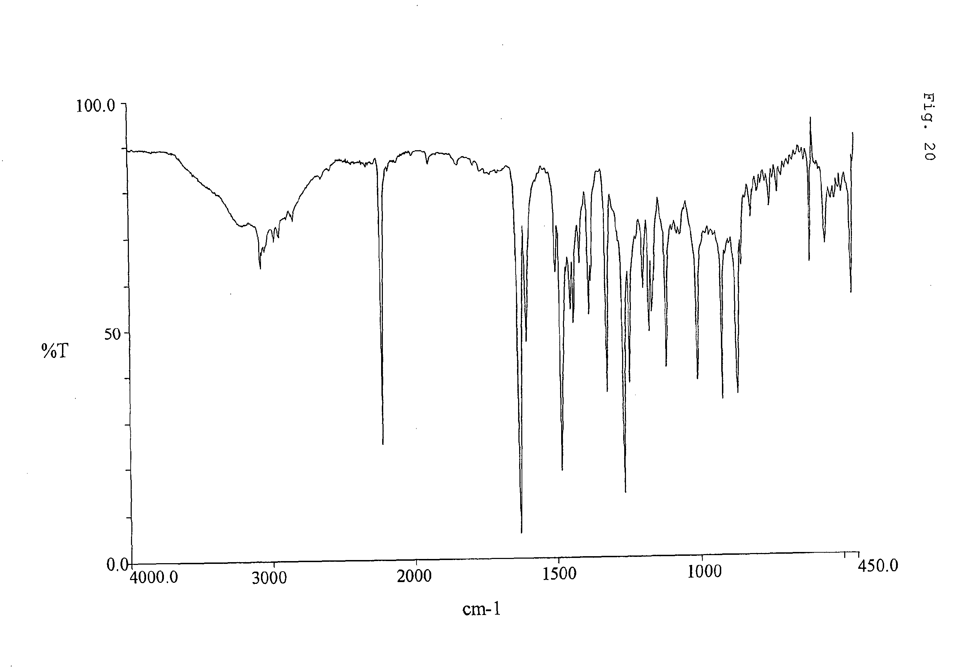

7.4 g of 2-methoxynaphthalene-3,6-dicarboxylic acid was suspended in 75 g of tetrahydrofuran and to this suspension, 14.3 g of thionyl chloride was added. The mixture was reacted at 45°C for one hour and excess thionyl chloride and solvent were distilled away. The residue was dissolved in 150 g of tetrahydrofuran and the mixture was heated to 45°C. The solution was added with ammonia gas and reacted for one hour and then the precipitated crystal was filtrated. 6.0 g of the resulting 2-methoxy-3,6-diaminocarbonylnaphthalene was suspended in 120 g of 1,2-dichlorobenzene and to this suspension, 4.1 g of phosphoryl chloride was added. The mixture was reacted at 140°C for one hour and then cooled to 80°C. The mixture was added with 150 g of water and stirred thoroughly. The precipitated crystal was filtrated, washed with water and methanol and dried to give 3.6 g of pale orange powder (decomposition point: 255°C). The infrared absorption spectrum (KBr method) of this compound is shown in Figure 20.

### Example 21

### Synthesis of 2-n-butoxy-3,6-dicyanonaphthalene

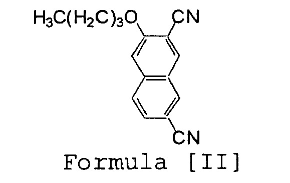

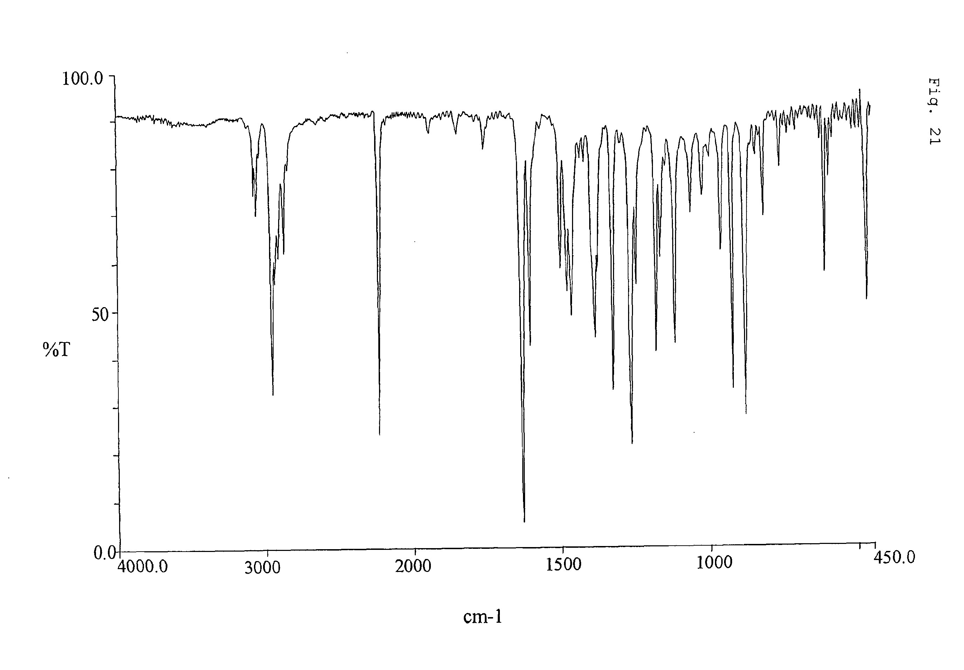

According to the same manner as Example 20 with the exception that 8.6 g of 2-n-butoxy-3,6-dicarboxylic acid was used instead of 7.4 g of 2-methoxynaphthalene-3,6-dicarboxylic acid, 3.1 g of white powder was obtained (melting point: 181°C, decomposition point: 265°C). The infrared absorption spectrum (KBr method) of this compound is shown in Figure 21.

### Example 22

### Synthesis of 2-n-octyloxy-3,6-dicyanonaphthalene

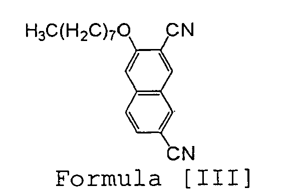

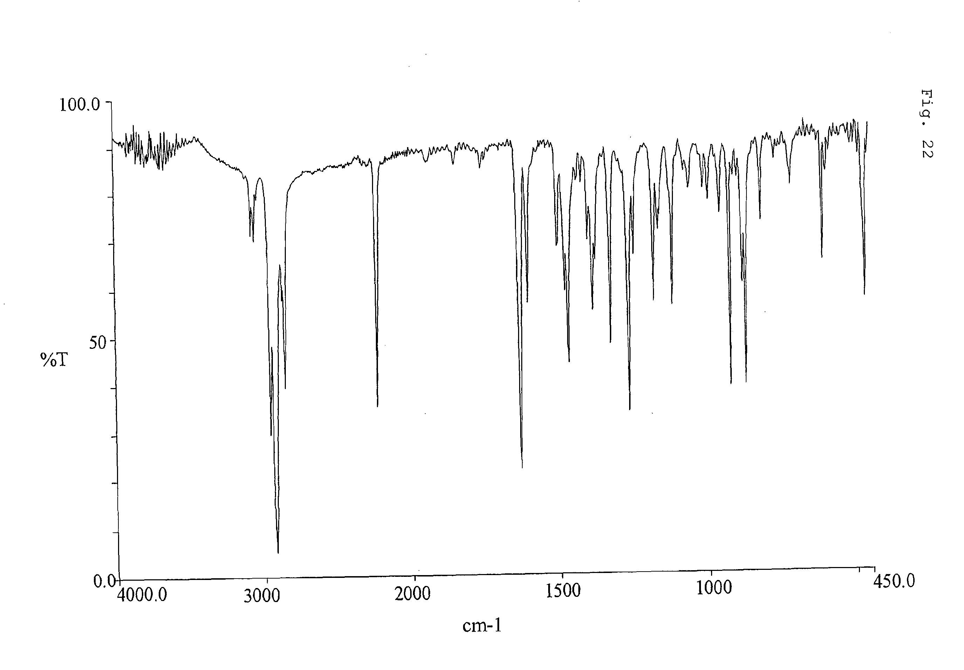

According to the same manner as Example 20 with the exception that 10.3 g of 2-n-octyloxynaphthalene-3,6-dicarboxylic acid was used instead of 7.4 g of 2-methoxynaphthalene-3,6-dicarboxylic acid, 4.4 g of white powder was obtained (melting point: 160°C, decomposition point: 280°C). The infrared absorption spectrum (KBr method) of this compound is shown in Figure 22.

### Example 23

### Synthesis of 2-n-dodecyloxy-3,6-dicyanonaphthalene

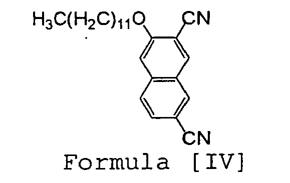

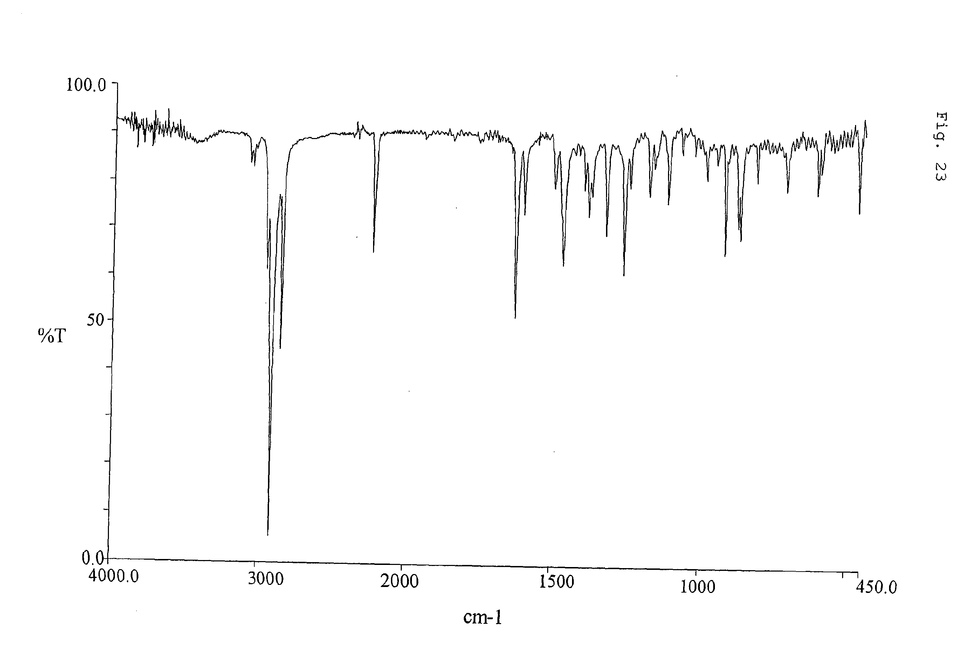

According to the same manner as Example 20 with the exception that 12.0 g of 2-n-dodecyloxynaphthalene-3,6-dicarboxylic acid was used instead of 7.4 g of 2-methoxynaphthalene-3,6-dicarboxylic acid, 5.8 g of white powder was obtained (melting point: 157°C, decomposition point: 297°C). The infrared absorption spectrum (KBr method) of this compound is shown in Figure 23.

### Example 24

### Synthesis of 2-hydroxy-3,6-dicyanonaphthalene

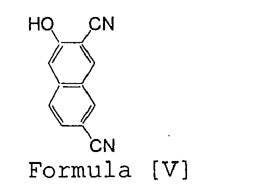

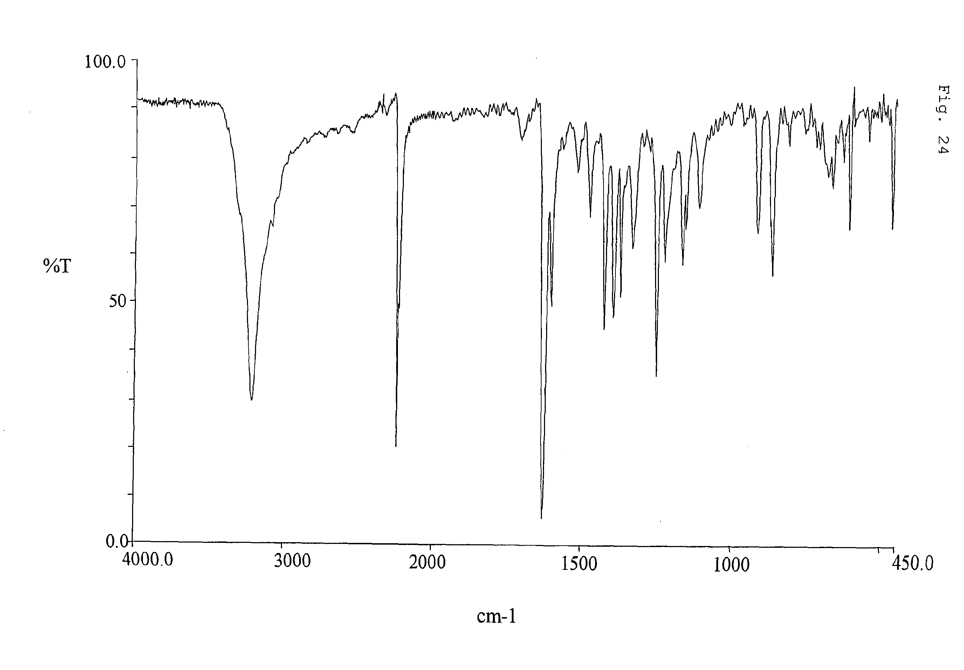

3.1 g of 2-methoxy-3,6-dicyanonaphthalene obtained in Example 20 was suspended in 100 g of benzene and to this suspension, 10 g of aluminum chloride was added. The mixture was reacted at 75°C for two hours and then cooled to 50°C. The mixture was added with 50 g of water and stirred thoroughly. The precipitated crystal was filtrated, washed with methanol and dried to give 2.5 g of white powder (decomposition point: 290°C). The infrared absorption spectrum (KBr method) of this compound is shown in Figure 24.

### Example 25

### Synthesis of 2-acetoxy-3,6-dicyanonaphthalene

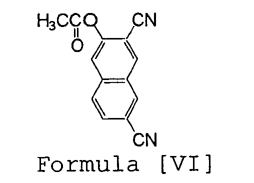

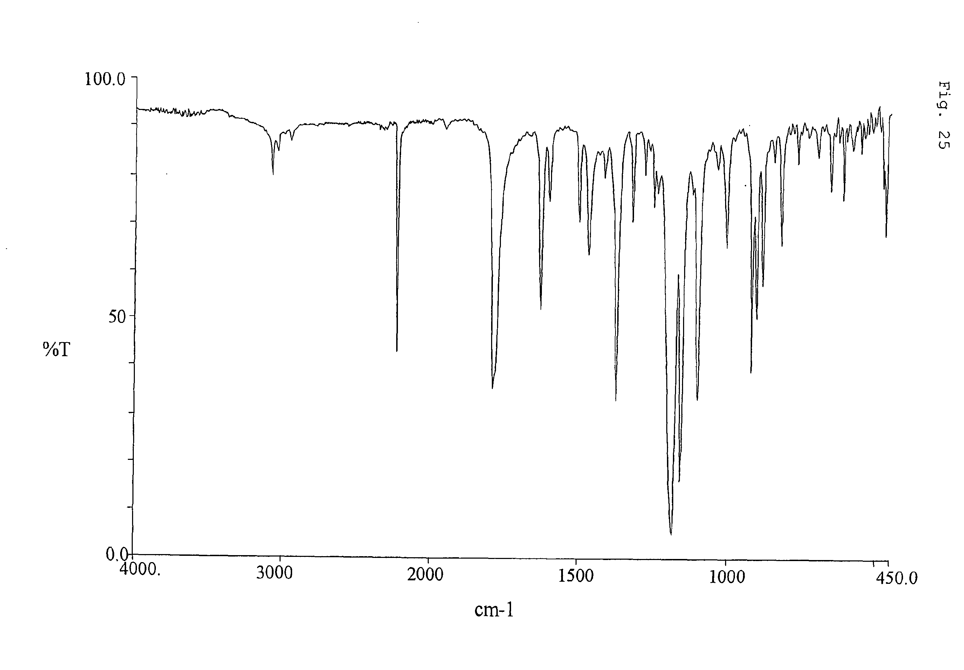

1.0 g of 2-hydroxy-3,6-dicyanonaphthalene obtained in Example 24 was suspended in the mixture consisting of 6 g of acetic anhydride and 4 g of glacial acetic acid and to this suspension, small amount of N,N-dimethylaminopyridine was added. The mixture was reacted at 70°C for two hours and poured into 20 g of water. The precipitated crystal was filtrated, washes with water and methanol and dried to give 1.0 g of pale orange powder (melting point: 185°C, decomposition point: 237°C). The infrared absorption spectrum (KBr method) of this compound is shown in Figure 25.

### Example 26

### Synthesis of 2-hydroxy-3,6-dicyanonaphthalene sodium salt

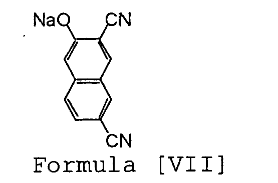

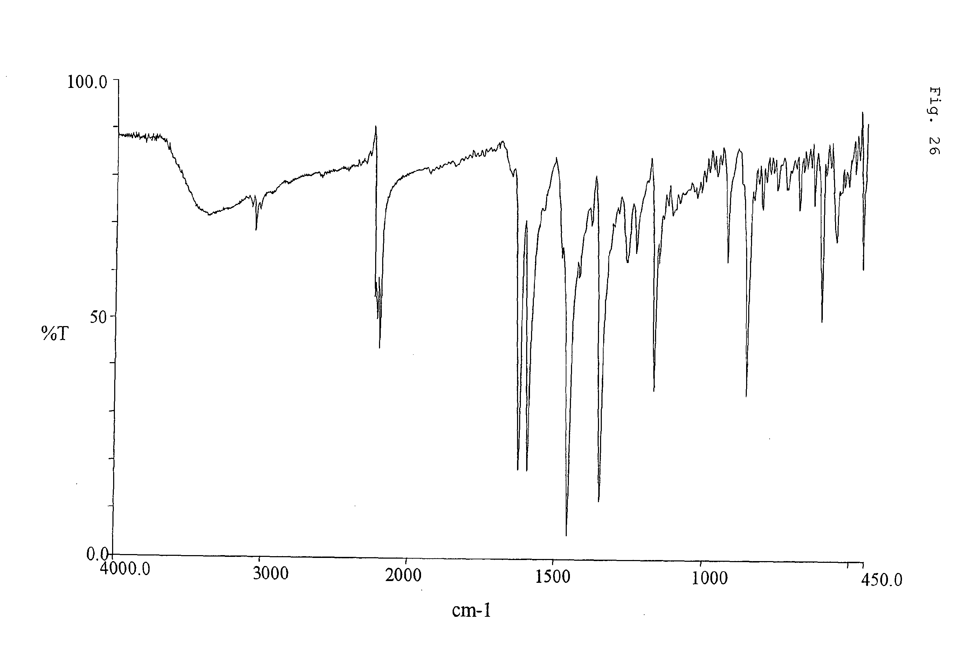

1.0 g of 2-hydroxy-3,6-dicyanonaphthalene obtained in Example 24 was suspended in 10 g of methanol and to this suspension, 1.0 g of 28 weight % solution of sodium methoxide in methanol was added dropwise. After the completion of the addition, the suspension became almost transparent solution. From the solution, a few amount of insoluble matter was removed by filtration and the filtrate was concentrated and dried to give 1.1 g of yellow powder (decomposition point: no lower than 500°C). The infrared absorption spectrum (KBr method) of this compound is shown in Figure 26.

### Example 27

### Synthesis of 2-benzyloxy-3,6-dicyanonaphthalene

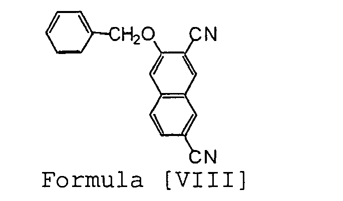

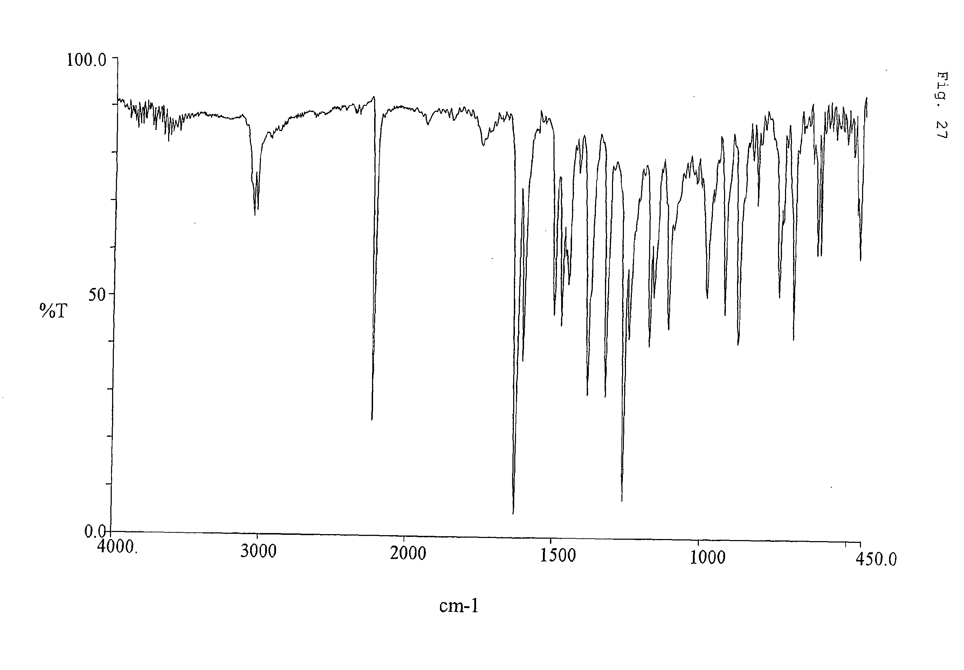

4.8 g of 2-benzyloxynaphthalene-3,6-dicarboxylic acid was suspended in 50 g of tetrahydrofuran and to this suspension, 7.2 g of thionyl chloride was added. The mixture was reacted at 45°C for one hour and then excess thionyl chloride and solvent were distilled away. The residue was dissolved in 50 g of tetrahydrofuran and the mixture was heated to 45°C. To this solution, ammonia gas was added and the mixture was reacted for one hour and the precipitated crystal was filtrated. 3.6 g of thus obtained 2-benzyloxy-3,6-diaminocarbonylnaphthalene was suspended in 60 g of 1,2-dichlorobenzene and to this suspension, 1.8 g of phosphoryl chloride was added. The mixture was reacted at 140°C for 3 hours and then cooled to 80°C. The mixture was added with 60 g of water and stirred thoroughly. The mixture was left to phase separation and the organic phase was isolated. To the organic phase, 100 g of hexane was added to precipitate the crystal. The crystal was filtrated, washed with methanol and dried to give 1.7 g of pale yellow powder (decomposition point: 278°C). The infrared absorption spectrum (KBr method) of this compound is shown in Figure 27.

### Example 28

### Synthesis of 2-methoxy-3-cyano-6-methoxycarbonylnaphthalene

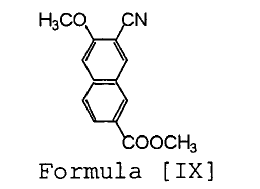

24.6 g of 2-hydroxy-6-methoxycarbonylnaphthalene-3-carboxylic acid was suspended in 300 g of tetrahydrofuran and to this suspension, 35.7 g of thionyl chloride was added. The mixture was reacted at 45°C for one hour and then excess thionyl chloride and solvent were distilled away. The residue was dissolved in 300 g of tetrahydrofuran and heated to 45°C. The mixture was added with ammonia gas and reacted for one hour. The precipitated crystal was filtrated.

14.7 g of thus obtained 2-hydroxy-3-aminocarbonyl-6-methoxycarbonylnaphthalene was dissolved in 150 g of N,N-dimethylformamide. To the solution, 8.0 g of 50 % aqueous potassium hydroxide and 11.1 g of methyl iodide were added. The mixture was reacted at room temperature for one day and poured into 300 g of water. The precipitated crystal was filtrated and dried.

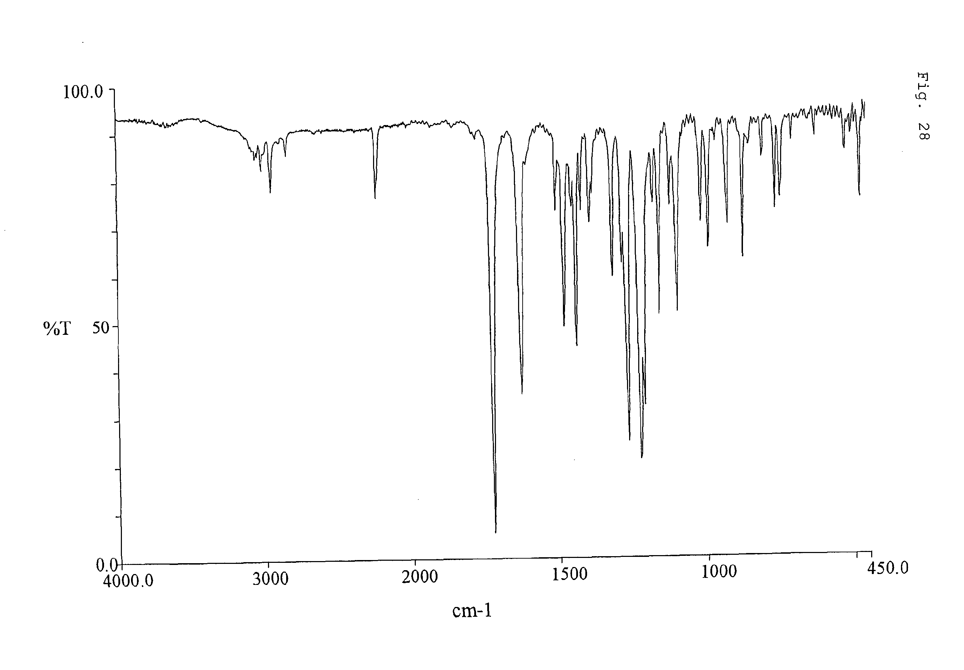

7.8 g of thus obtained 2-methoxy-3-aminocarbonyl-6-methoxycarbonylnaphthalene was suspended in 80 g of 1,2-dichlorobenzene and to this suspension, 2.8 g of phosphoryl chloride was added. The mixture was reacted at 140°C for two hours and then cooled to 80°C. The mixture was added with 80 g of water and stirred thoroughly. The precipitated crystal was filtrated, washer with methanol and dried to give 5.8 g of white powder (melting point: 202°C, decomposition point: 229°C). The infrared absorption spectrum (KBr method) of this compound is shown in Figure 28.

### Example 29

### Synthesis of 2-methoxy-3-cyanonaphthalene-6-carboxylic acid

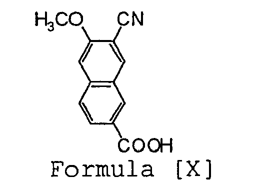

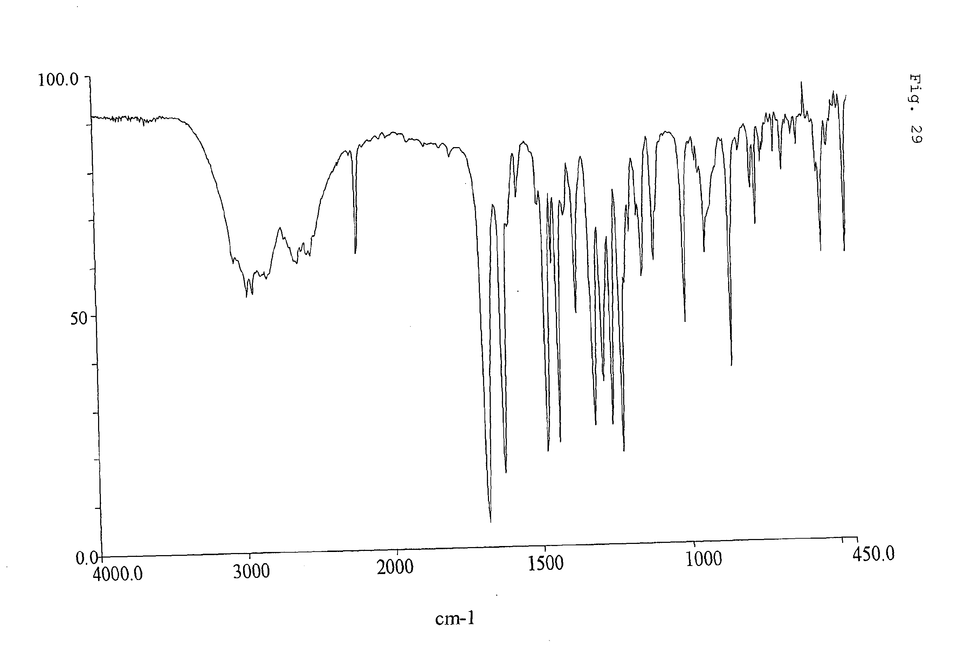

5.0 g of 2-methoxy-3-cyano6-methoxycarbonylnaphthalene obtained in Example 28 was suspended in 50 g of methanol. The suspension was added with 25 g of 10 % aqueous sodium hydroxide and reacted at 65°C for two hours. After that, the mixture was subjected to neutralization and acid crystallization by addition of hydrochloric acid. The precipitated crystal was filtrated, washed with water and methanol and dried to give 4.2 g of white powder (decomposition point: 301°C). The infrared absorption spectrum (KBr method) of this compound is shown in Figure 29.

### Example 30

### Synthesis of 2-methoxy-3-cyano-6-aminocarbonylnaphthalene

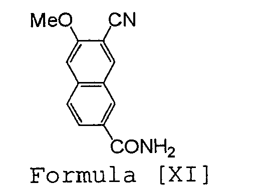

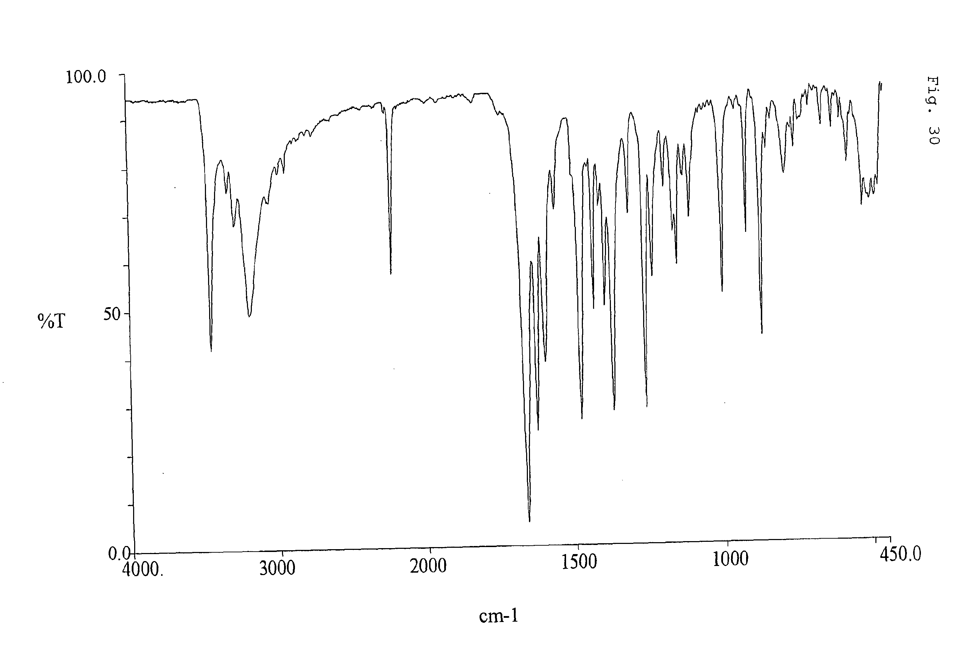

5 g of 2-methoxy-3-cyanonaphthalene-6-carboxylic acid obtained in Example 29 was suspended in 50 g of tetrahydrofuran and to this suspension, 5.2 g of thionyl chloride was added. The mixture was reacted at 45°C for one hour and then excess thionyl chloride and solvent were distilled away. The residue was dissolved in 50 g of tetrahydrofuran and heated to 45°C. The solution was added with ammonia gas and reacted for one hour. After that, the precipitated crystal was filtrated, washed with water and methanol and dried to give 3.7 g of white powder (decomposition point: 300°C). The infrared absorption spectrum (KBr method) of this compound is shown in Figure 30.

### Example 31

### Synthesis of 2-methoxy-3-cyano-6-(2-chlorophenylureidocarbonyl)naphthalene

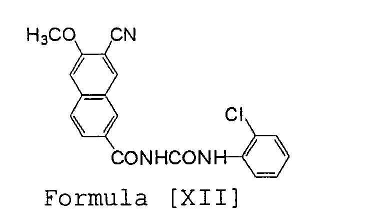

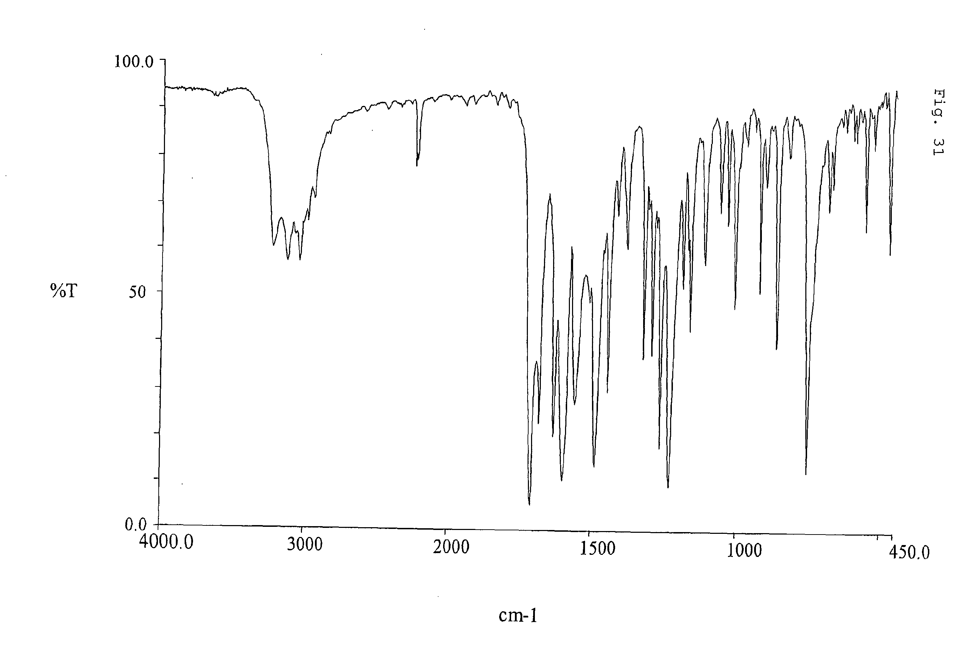

4.0 g of 2-methoxy-6-(2-chlorophenylureidocarbonyl)naphthalene-3-carboxylic acid was suspended in 80 g of tetrahydrofuran and to this suspension, 2.4 g of thionyl chloride was added. The mixture was reacted at 45°C for one hour and then excess thionyl chloride and solvent were distilled away. The residue was dissolved in 80 g of tetrahydrofuran and heated to 45°C. The solution was added with ammonia gas and reacted for one hour. The precipitated crystal was filtrated. 2.4 g of thus obtained 2-methoxy-3-aminocarbonyl-6-(2-chlorophenylureidocarbonyl)naphthalene was suspended in 60 g of 1,2-dichlorobenzene and to this suspension, 1.1 g of phosphoryl chloride was added. The mixture was reacted at 140°C for three hours and then cooled to 80°C. The mixture was then added with 60 g of water and stirred thoroughly. The precipitated crystal was filtrated, washed with methanol and dried to give 1.4 g of white crystal (decomposition point: 271°C). The infrared absorption spectrum (KBr method) of this compound is shown in Figure 31.

### Example 32-1

### Synthesis of 2-methoxy-3-cyano-6-(benzo-1',3'-thiazol-2'-yl)naphthalene (1)

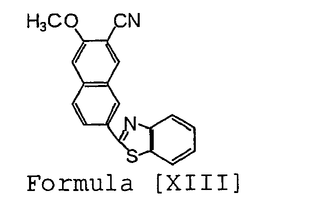

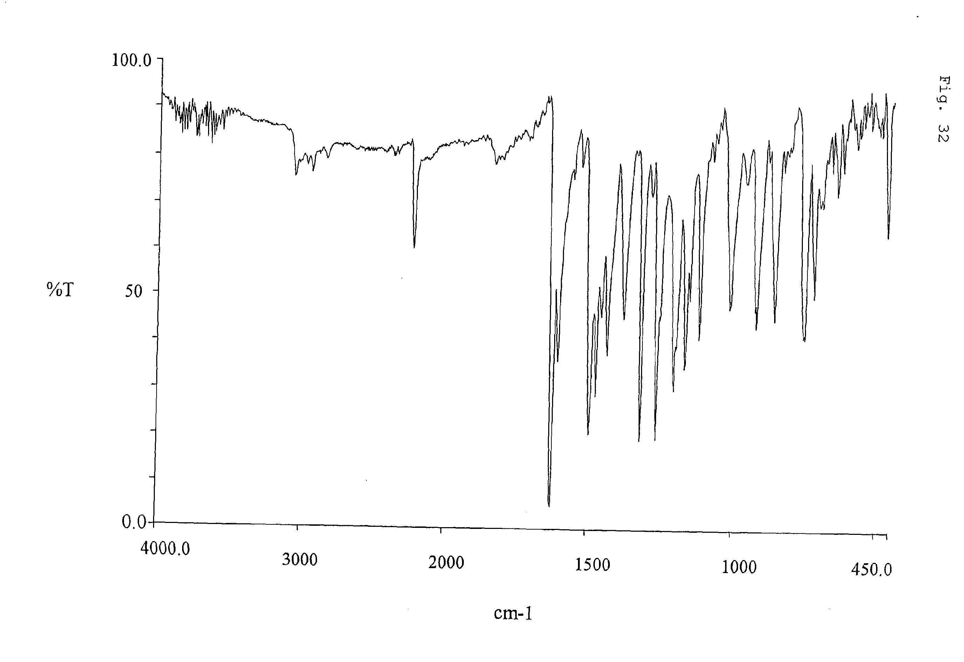

3.4 g of 2-methoxy-6-(benzo-1',3'-thiazol-2'-yl)naphthalene-3-carboxylic acid was suspended in 100 g of tetrahydrofuran and to this suspension, 2.4 g of thionyl chloride was added. The mixture was reacted at 45°C for one hour and then excess thionyl chloride and solvent were distilled away. The residue was dissolved in 80 g of tetrahydrofuran and the solution was heated to 45°C. The solution was added with ammonia gas and reacted for one hour. The precipitated crystal was filtrated. 2.4 g of thus obtained 2-methoxy-3-aminocarbonyl-6-(benzo-1',3'-thiazol-2'-yl)naphthalene was suspended in 60 g of 1,2-dichlorobenzene and to this suspension, 1.1 g of phosphoryl chloride was added. The mixture was reacted at 140°C for three hours and then cooled to 80°C. Then the mixture was added with 60 g of water and stirred thoroughly. The precipitated crystal was filtrated, washed with methanol and dried to give 2.0 g of yellow powder (melting point: 220°C, decomposition point: 340°C). The infrared absorption spectrum (KBr method) of this compound is shown in Figure 32.

### Example 32-2

### Synthesis of 2-methoxy-3-cyano-6-(benzo-1',3'-thiazol-2'-yl)naphthalene (2)

The compound obtained in Example 32-1 may be synthesized by the following procedure.

1.0 g of 2-methoxy-3-cyanonaphthalene-6-carboxylic acid obtained in Example 29 and 1.0 g of 2-aminobenzenethiol were suspended in 25 g of sulfolane and to this suspension, 0.8 g of phosphorous trichloride was added. The mixture was reacted at 140°C for two hours and then cooled to room temperature. The mixture was added with 50 g of methanol and filtrated. The resulting crystal was washed with warm water and methanol and dried to give 1.1 g of yellow powder.

### Example 33

### Synthesis of 2-methoxy-3-phenylaminocarbonyl-6-cyanonaphthalene

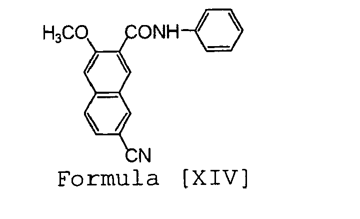

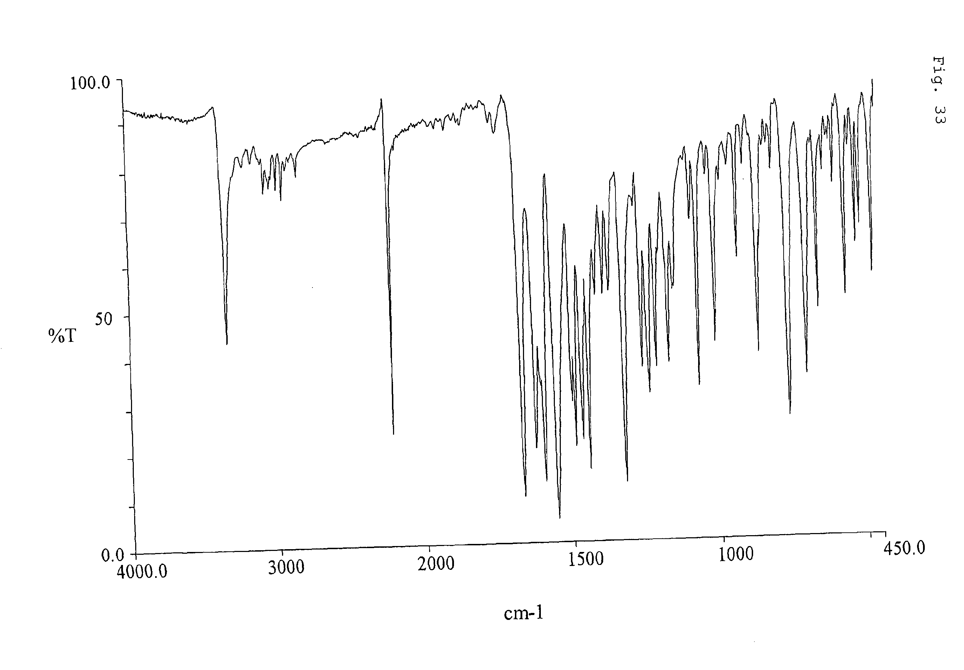

4.6 g of 2-methoxy-3-phenylaminocarbonylnaphthalene-6-carboxylic acid was suspended in 45 g of tetrahydrofuran and to this suspension, 3.6 g of thionyl chloride was added. The mixture was reacted at 45°C for one hour and then excess thionyl chloride and solvent were distilled away. The residue was dissolved in 50 g of tetrahydrofuran and the solution was heated to 45°C. The solution was added with ammonia gas and reacted for one hour. The precipitated crystal was filtrated. 3.0 g of thus obtained 2-methoxy-3-phenylaminocarbonyl-6-aminocarbonylnaphthalene was suspended in 40 g of 1,2-dichlorobenzene and to this suspension, 1.0 g of phosphoryl chloride was added. The mixture was reacted at 140°C for one hour and then cooled to 80°C. The mixture was added with 50 g of water and stirred thoroughly. The precipitated crystal was filtrated, washed with water and methanol and dried to give 1.8 g of white powder (melting point: 201°C, decomposition point: 319°C). The infrared absorption spectrum (KBr method) of this compound is shown in Figure 33.

## Claims

1. An aminocarbonylnaphthol derivative represented by formula (1): 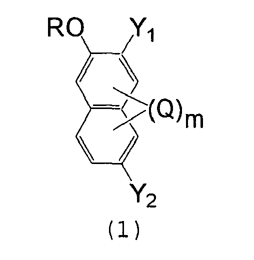 wherein each Y₁ and Y₂ is independently selected from the group consisting of aminocarbonyl group, a group represented by formula (2), a group represented by formula(3), a group represented by formula (4) and carboxyl group, provided that at least one of Y₁ and Y₂ represents aminocarbonyl group;
― (CONH)ₙ―X₁ (2)
―CO―O―X₂ (3) 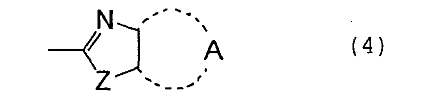
wherein n is an integer of 1 or 2;
X₁ is selected from the group consisting of an optionally branched, optionally substituted, saturated or unsaturated aliphatic group having 1-20 carbon atoms, an optionally substituted aromatic group and an optionally substituted heterocyclic group having conjugated double bonds;
X₂ is an optionally branched, saturated or unsaturated aliphatic group having 1-6 carbon atoms;
A is an optionally substituted aromatic group or an optionally substituted heterocyclic group having conjugated double bonds;
Z is selected from the group consisting of -O-, - S- and -NH-;
Q is selected from the group consisting of an optionally branched alkyl group having 1-6 carbon atoms, an optionally branched alkoxy group having 1-6 carbon atoms, halogen atom, nitro group and nitroso group;
m is an integer of 0-3;
R is selected from the group consisting of hydrogen atom, an alkaline metal, an optionally branched and optionally substituted alkyl group having 1-20 carbon atoms, an optionally branched and optionally substituted acyl group having 2-20 carbon atoms and phenylalkyl group.

2. The aminocarbonylnaphthol derivative according to claim 1, wherein R is selected from the group consisting of an optionally branched and optionally substituted alkyl group having 1-20 carbon atoms and phenylalkyl group.

3. A process for preparing an aminocarbonylnaphthol derivative represented by formula (6) comprising converting a carboxyl group of a naphthol derivative having carboxyl group represented by formula (5) to an acid halide and reacting the resulting acid halide of the naphthol derivative with ammonia: 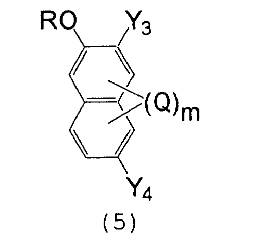 wherein each Y₃ and Y₄ is independently selected from the group consisting of carboxyl group and groups represented by formulae (2), (3) and (4) defined in claim 1, provided that at least one of Y₃ and Y₄ is carboxyl group; R, Q and m are the same as defined in claim 1; 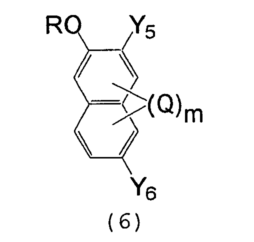 wherein each Y₅ and Y₆ is independently selected from the group consisting of aminocarbonyl group and groups represented by formulae (2), (3) and (4) defined in claim 1, provided that at least one of Y₅ and Y₆ is aminocarbonyl group; R, Q and m are the same as defined in claim 1.

4. A cyanonaphthol derivative represented by formula (7) : 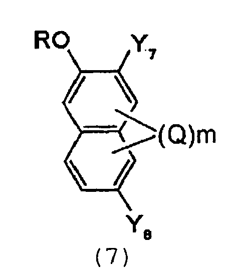 wherein each Y₇ and Y₈ is independently selected from the group consisting of cyano group, groups represented by the formulae (2), (3) and (4) defined in claim 1, carboxyl group and aminocarbonyl group, provided that at least one of Y₇ and Y₈ is cyano group; Q, R and m are the same as defined in claim 1; and a salt thereof.

5. A cyanonaphthol derivative represented by formula (8): 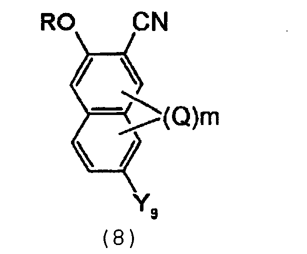 wherein Yg is selected from the group consisting of cyano group, groups represented by formulae (2), (3) and (4) defined in claim 1, carboxyl group and aminocarbonyl group; R, Q and m are the same as defined in claim 1; and a salt thereof.

6. A process for preparing a cyanonaphthol derivative represented by formula (10) comprising converting a carboxyl group of a naphthol derivative having carboxyl group represented by formula (9) to aminocarbonyl group, and reacting the resulting naphthol derivative having aminocarbonyl group with a dehydrating agent: 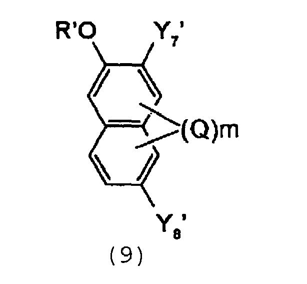 wherein each Y₇' and Y₈' is independently selected from the group consisting of carboxyl group and groups represented by formulae (2), (3) and (4) defined in claim 1, provided that at least one of Y₇' and Y₈' is carboxyl group;
R' is selected from the group consisting of an optionally branched and optionally substituted alkyl group having 1-20 carbon atoms and phenylalkyl group;
Q and m are the same as defined in claim 1; 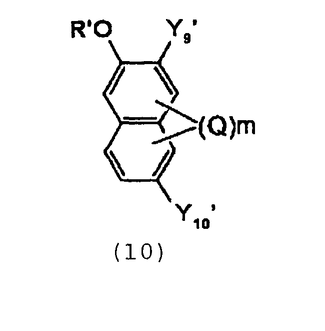 wherein each Y₉' and Y₁₀' is independently selected from the group consisting of cyano group and groups represented by formulae (2), (3) and (4) defined in claim 1, provided that at least one of Y₉' and Y₁₀' is cyano group; R', Q and m are the same as defined in claim 1.

7. The process for preparing the cyanonaphthol derivative of claim 6 wherein said dehydrating agent is phosphoryl chloride.
